(19) Europäisches Patentamt
European Patent Office
Office européen des brevets

(11) Numéro de publication: **0 363 275 A1**

(12) **DEMANDE DE BREVET EUROPEEN**

(21) Numéro de dépôt: **89402730.9**

(22) Date de dépôt: **03.10.89**

(51) Int. Cl.5: **C07H 15/04 , A61K 47/00 , A61K 49/02**

(30) Priorité: **05.10.88 FR 8813026**

(43) Date de publication de la demande: **11.04.90 Bulletin 90/15**

(84) Etats contractants désignés: **AT BE CH DE ES FR GB GR IT LI LU NL SE**

(71) Demandeur: **IRE-CELLTARG S.A.**

**B-6220 Fleurus(BE)**

(72) Inventeur: **Mary, Anne**
**15, rue Joseph Huberty**
**B-4171 Combain-La-Tour(BE)**
Inventeur: **Falmagne, Jean-Bernard**
**19, Montagne d'Aisemont**
**B-1300 Wavre(BE)**
Inventeur: **Trouet, André**
**29, Predikherenberg**
**B-3009 Winksele-Herent(BE)**

(74) Mandataire: **Warcoin, Jacques et al**
**Cabinet Régimbeau 26, avenue Kléber**
**F-75116 Paris(FR)**

(54) **Vecteurs synthétiques de faible poids moléculaire spécifiques de récepteurs à sucre, conjugués en comportant et procédés de préparation.**

(57) La présente invention a pour objet un vecteur synthétique consistant dans un composé chimique de faible poids moléculaire, en particulier inférieur à 5000, présentant une affinité pour les cellules exposant à leur membrane plasmique des récepteurs reconnaissant spécifiquement des résidus sucrés consistant en des cycles glycosidiques de saccharides, caractérisé en ce qu'il présente une structure chimique de base sur laquelle sont fixés plusieurs cycles glycosidiques de saccharides espacés de telle manière que chaque site de reconnaissance de ces résidus sucrés du recepteur soit susceptible d'être occupé.

la présente invention a également pour objet l'utilisation de ces vecteurs comme agent d'imagerie ou des conjugués de ce type de vecteur avec un médicament, notamment choisi parmi les substances antitumorales, antivirales et antiparasitaires, ainsi que des procédés de préparation desdits vecteurs.

EP 0 363 275 A1

# VECTEURS SYNTHETIQUES DE FAIBLE POIDS MOLECULAIRE SPECIFIQUES DE RECEPTEURS A SUCRE,CONJUGUES EN COMPORTANT ET PROCEDES DE PREPARATION

La présente invention concerne des vecteurs synthétiques consistant dans un composé chimique de faible poids moléculaire, en particulier inférieur à 5000, présentant une affinité pour les cellules exposant à leur membrane plasmique des récepteurs reconnaissant spécifiquement des résidus sucrés consistant en des cycles glycosidiques de saccharides.

Ces vecteurs sont utiles comme agents de pilotage en général, c'est-à-dire aussi bien à des fins de thérapie ciblée lorsqu'ils sont couplés à un médicament qu'en imagerie médicale lorsqu'ils sont associés à une molécule de marquage détectable par exemple par radioactivité.

La présente invention concerne également des conjugués du type vecteur-médicament comportant lesdits vecteurs.

Le concept, consistant à rendre plus sélectif un médicament par couplage à des vecteurs spécifiques de cellules-cibles, fut formulé pour la première fois par EHRLICH en 1906. Cette approche originale s'est concrétisée par la découverte, à la surface de certaines cellules, de molécules particulières capables d'interagir spécifiquement avec des macromolécules présentes dans les milieux extracellulaires. C'est ainsi que de nombreux travaux ont montré que l'association d'une substance active à une macromolécule vectrice transportant l'agent thérapeutique de son site d'application vers son site d'action réduit son interaction avec les cellules normales, ce qui diminue sa toxicité et augmente son index thérapeutique (TROUET et al., 1972 ; GREGORIADIS, 1979 ; TROUET et al., 1981 ; PIRSON, thèse de doctorat, 1984).

Dans le cas particulier du foie, le transport spécifique de substances médicamenteuses a pu être envisagé grâce à la mise en évidence par ASHWELL et MORELL (1974) de récepteurs à asialoglycoprotéine au niveau de la membrane sinusoïdale des hépatocytes. Ces récepteurs, dont le rôle physiologique reste obscur, reconnaissent les résidus galactoses ou N-acétylgalactosamine en position terminale des glycoprotéines. Ils ont été estimés à 250.000 par cellule et la constante de dissociation ligand-récepteur est d'environ $2x10^{-8}$ M (BAENZIGER et al., 1980).

D'un point de vue cellulaire, après fixation de l'asialoglycoprotéine à son récepteur, le complexe ligand-récepteur est intériorisé dans des vésicules intracellulaires acides appelées endosomes, dans lesquelles le ligand se dissocie du récepteur. Le ligand libre est ensuite acheminé vers les lysosomes où il est finalement dégradé.

MASQUELIER (thèse de doctorat, 1981) a montré que le couplage d'un médicament à une asialoglycoprotéine par le biais d'un lien covalent ou " bras", sensible aux hydrolases lysosomiales, permet, d'une part, l'endocytose spécifique du conjugué médicament-transporteur par des hépatocytes et, d'autre part, la restauration intracelllulaire du médicament sous une forme active.

L'efficacité thérapeutique du traitement semble être fortement dépendante des caractéristiques du vecteur employé.

La présente invention, qui s'inscrit dans ce contexte, a pour but, d'une manière générale, la conception d'un nouveau type de vecteur spécifique de cellules exposant des récepteurs à sucres, en particulier les cellules parenchymateuses du foie.

Les asialoglycoprotéines et néoglycoprotéines constituent jusqu'à présent l'essentiel des vecteurs macromoléculaires employés.

En principe, un médicament couplé à une asialoglycoprotéine ou une néoglycoprotéine sera sélectivement dirigé vers le foie et plus spécifiquement vers les hépatocytes où la substance médicamenteuse sera libérée et pourra exercer son activité thérapeutique.

En particulier, un agent antimalarique actif sur les formes exoérythrocytaires de la malaria, tel que la primaquine, a été couplé à de l'asialofétuine et de l'albumine galactosylée (PIRSON, thèse de doctorat). Ce conjugué est effectivement reconnu par les hépatocytes et s'est avéré plus actif que la primaquine libre sur les formes exoérythrocytaires de la malaria murine.

Cependant, bien qu'efficaces, ces vecteurs macromoléculaires présentent certains inconvénients. En effet, leur poids moléculaire élevé entraîne

- une excrétion rénale difficile, voire même impossible, entraînant la persistance du conjugué dans la circulation, ce qui peut favoriser une toxicité secondaire non désirée,
- un risque d'immunogénicité accru lors du couplage du médicament ou lors d'administrations répétées du conjugué.

Les désavantages possibles engendrés par la taille des vecteurs macromoléculaires devraient pouvoir être contournés par l'utilisation de glycopeptides naturels, caractérisés par un faible poids moléculaire.

Il est en effet maintenant bien établi que c'est par le biais de résidus galactoses terminaux que les

asialoglycoprotéines sont reconnues spécifiquement, puis endocytées par les hépatocytes.

BAENZIGER et al. (1979) ont réussi à isoler et purifier un glycopeptide à partir d'une glycoprotéine naturelle, la fétuine.

Cependant, malgré les résultats globalement positifs obtenus sur cellules en culture, il est apparu
- qu'il était fastidieux et coûteux de purifier le glycopeptide en quantité importante ;
- qu'il était difficile, voire même impossible, d'obtenir le glycopeptide de façon homogène et reproductible ; ceci pouvant s'expliquer par la grande hétérogénéité des structures glycaniques caractérisant la plupart des glycoprotéines naturelles.

Afin d'améliorer les caractéristiques de tels vecteurs et, notamment, afin d'obtenir de façon reproductible un produit homogène stable et bien caractérisé, on a selon la présente invention synthétisé, par voie chimique, différents dérivés comportant des résidus de sucres, tels que des galactoses en position terminale.

En 1984, KEMPEN et al., ont réalisé la synthèse chimique d'une structure glycanique simple, à partir d'amino 2-(hydroxyméthyl)-1,3-propanediol (Tris) et de galactose.

Des études in vitro ont montré cependant que ce dérivé ne présentait qu'une affinité faible pour le récepteur. Le fait pourrait, en partie, s'expliquer par un arrangement spatial inadéquat résultant de la trop courte distance séparant les trois résidus de galactoses.

En effet, il est apparu que pour être efficacement reconnue puis endocytée par les hépatocytes, la partie glycanique d'une asialoglycoprotéine doit comporter au moins trois galactoses en position terminale.

De plus, certains critères d'arrangements spatiaux doivent être respectés ; les galactoses terminaux devant se fixer simultanément aux 3 sites de fixation du récepteur, distants respectivement de 15, 22 et 25 Å.

C'est pourquoi, la présente invention a pour objet un vecteur synthétique consistant dans un composé chimique de faible poids moléculaire, en particulier inférieur à 5000, présentant une affinité pour les cellules exposant à leur membrane plasmique des récepteurs reconnaissant spécifiquement des résidus sucrés consistant en des restes de cycles glycosidiques de saccharides, vecteur caractérisé en ce qu'il présente une structure chimique de base sur laquelle sont fixés plusieurs résidus sucrés consistant en des cycles glycosidiques de saccharides, espacés de telle manière que chaque site de reconnaissance de ces résidus sucrés, exposés à la membrane plasmique du récepteur, soit susceptible d'être occupé par lesdits résidus sucrés du vecteur.

Cette structure chimique de base est constituée de façon générale par un reste hydrocarboné.

En particulier et de façon non limitative, on peut avoir recours comme structure chimique de base à un acide aminé ou un peptide de 2 à 7 acides aminés. Ce type de structure offre entre autres l'avantage de pouvoir être directement couplé à un médicament lorsque le vecteur est utilisé à cet effet. Les liens entre le médicament et le vecteur sinon doivent être stables dans le serum et hydrolysables dans les lysosomes.

Dans un mode particulier de réalisation, on a découvert, selon l'invention, qu'une combinaison par exemple entre un acide bi ou polyfonctionnel, comme l'acide aspartique, et plusieurs molécules de Tris trigalactoses donnant accès à des structures glycaniques comportant plus de 3 galactoses terminaux se traduisait par une meilleure spécificité des vecteurs. En effet, dans ces structures complexes, l'augmentation de la distance entre certains galactoses correspond à l'exigence spatiale requise par la configuration du récepteur.

Ce type de vecteur synthétique permet
- de diminuer fortement la réaction immunitaire de par son poids moléculaire faible ;
- une élimination rénale rapide qui évite tout effet secondaire non désiré ;
- une incorporation aisée dans des polymères biodégradables servant de réservoir à relargage progressif.

Cette synthèse par voie chimique permet en outre d'obtenir facilement de façon reproductible et stable un produit homogène et bien caractérisé.

De plus, contrairement aux vecteurs naturels cités dont la préparation nécessite la manipulation de fractions sanguines, cette nouvelle approche élimine tout risque, si faible soit-il d'une contamination par des agents pathogènes sanguins. Elle est par conséquent préférable pour une application clinique.

L'affinité du vecteur peut être modulée par la présence d'autres sucres que le galactose en position terminale, notamment la N-acétylgalactosamine connue pour sa haute affinité intrinsèque pour le récepteur.

Dans une autre approche, l'utilisation du lactose, disaccharide constitué du glucose et de galactose, permet d'augmenter la distance entre les galactoses terminaux. Ce qui a pour conséquence d'accroître l'affinité du composé pour le récepteur.

On peut également selon l'invention accroître dans la structure du vecteur la distance entre les galactoses terminaux en intercalant des restes hydrocarbonés divalents, tels que des restes d'acides aminés mais de manière non limitative, entre les cycles glycosidiques et l'extrémité hydroxyle du groupe

Tris.

L'emploi de ce vecteur n'est pas restrictif quant à la substance à piloter. Il peut en effet être utilisé pour cibler des substances anticancéreuses par exemple dans le cas des hépatomes, ou encore des substances antivirales par exemple dans le cas d'infection des cellules hépatiques par le virus de l'hépatite B.

Dans tous ces cas, le ciblage se fait par le biais du récepteur à asialoglycoprotéines présent au niveau de la membrane sinusoïdale des hépatocytes.

Ce type de vecteur n'est pas limité au pilotage de médicaments mais il peut également être utilisé notamment à des fins d'imagerie médicale lorsqu'il est associé à un élément de marquage, par exemple radioactif.

L'emploi de ce vecteur n'est pas non plus restrictif quant au type cellulaire que l'on veut atteindre. Les cellules du système réticuloendothélial, par exemple, exposent à leur membrane plasmique des récepteurs reconnaissant spécifiquement des résidus mannoses terminaux (STAHL et al., 1978 et 1980).

Il est dès lors tout à fait possible, selon l'invention, de synthétiser un vecteur possédant des mannoses comme sucres terminaux de façon à diriger sélectivement une substance médicamenteuse vers les cellules du système réticulo-endothélial.

D'autre part, on sait également que les macrophages possèdent des récepteurs à mannoses.

De façon tout à fait générale, ce type de vecteur peut être utilisé pour cibler n'importe quel type cellulaire qui exprimerait, au niveau de sa membrane plasmique, une molécule spécialisée dans la reconnaissance d'un résidu de sucre particulier.

Plus précisément, la présente invention a donc pour objet un vecteur synthétique consistant dans un composé chimique de faible poids moléculaire, en particulier inférieur à 5000, présentant une affinité pour les cellules exposant à leur membrane plasmique des récepteurs reconnaissant spécifiquement des résidus sucrés consistant en des cycles glycosidiques de saccharides, caractérisé en ce qu'il présente une structure chimique de base constituée par un acide aminé ou un peptide de 2 à 7 acides aminés, identiques ou différents, avec au moins deux fonctions carboxyles et une fonction amine libres, la ou certaines des fonction(s) carboxyle(s) libre(s) de l'acide aminé ou du peptide étant liée(s) par un lien amide à l'amine d'un groupe chimique doté en outre d'une fonction carboxyle, tel qu'un acide amino-alcanoïque, groupe faisant office de bras de liaison entre l'acide aminé ou le peptide et un groupe Tris, ladite fonction carboxyle du bras étant liée par un lien amide à l'amine du groupe Tris, ce dernier ayant certaines de ses fonctions hydroxy, transformées en fonctions éther par un reste de cycle glycosidique de saccharides déshydroxylé en position 1, de telle sorte que le vecteur dans son ensemble présente au moins autant de restes de cycles glycosidiques de saccharides que le récepteur n'a de site de reconnaissance de tels restes sucrés, et que ces restes de cycles glycosidiques de saccharides soient suffisamment espacés pour qu'une interaction puisse s'établir avec lesdits sites du récepteur.

Il apparaît qu'une telle structure présente une flexibilité suffisante pour que statistiquement, au cours de ses oscillations, tous les sites de reconnaissance du récepteur puissent être en correspondance interactive avec les restes sucrés du vecteur pour autant que ces restes soient en nombre suffisant, ce qui est une caractéristique fonctionnelle revendiquée selon l'invention pour ces vecteurs.

Dans un mode de réalisation de l'invention, des restes hydrocarbonés divalents sont intercalés entre des restes de cycles glycosidiques et des fonctions hydroxyle de groupe Tris ainsi étherifié par lesdits restes hydrocarbonés.

Par exemple, lesdits restes hydrocarbonés intercalés sont des restes $-CH_2-CH_2-O-$. Pour ce faire, on fait réagir de l'epoxyde d'éthylène sur les fonctions hydroxyle du groupe Tris lequel groupe Tris se transforme en triol terminé par des groupes éthanol dont on étherifie ensuite les fonctions hydroxyle par des restes de cycles glycosidiques.

Avantageusement, la ou une des fonctions amine libre(s) de l'acide aminé ou du peptide est protégée par un groupe protecteur de fonction amine, tel que le groupe carbobenzoxy (Cbz).

Cette fonction déprotégée permettra le couplage à une substance médicamenteuse éventuellement via un bras de liaison constitué d'un reste chimique divalent stable dans le plasma et hydrolysable dans les lysosomes selon des procédés faisant partie de l'état de la technique.

De préférence, les vecteurs, selon l'invention, présentant une structure chimique de base constituée d'acides aminés, comporteront deux ou trois acides aminés.

Une telle structure permet ainsi d'obtenir des composés bi (avec deux groupes Tris) ou de préférence Tri-antennés (avec trois groupes Tris) et jusqu'à neuf résidus sucrés avec une fonction amine disponible pour le couplage.

On peut citer comme bras chimique, entre une fonction carboxyle libre d'un acide aminé ou du peptide et un groupe Tris, un reste divalent d'un acide amino-alcanoïque de formule

$$-NH-(CH_2)_n-\underset{\underset{O}{\|}}{C}-$$

où n est compris entre 2 et 12.

On peut citer plus particulièrement le cas de l'acide 6-amino-hexanoïque où n = 5.

De façon appropriée, l'un au moins des acides aminés constituant ladite structure chimique de base sera un acide polyfonctionnel notamment choisi parmi l'acide aspartique et l'acide glutamique.

De préférence, les acides aminés de la structure chimique de base seront choisis parmi l'acide aspartique et l'acide glutamique.

A titre illustratif, on peut citer, comme vecteur selon l'invention, des composés chimiques répondant aux formules développées suivantes :

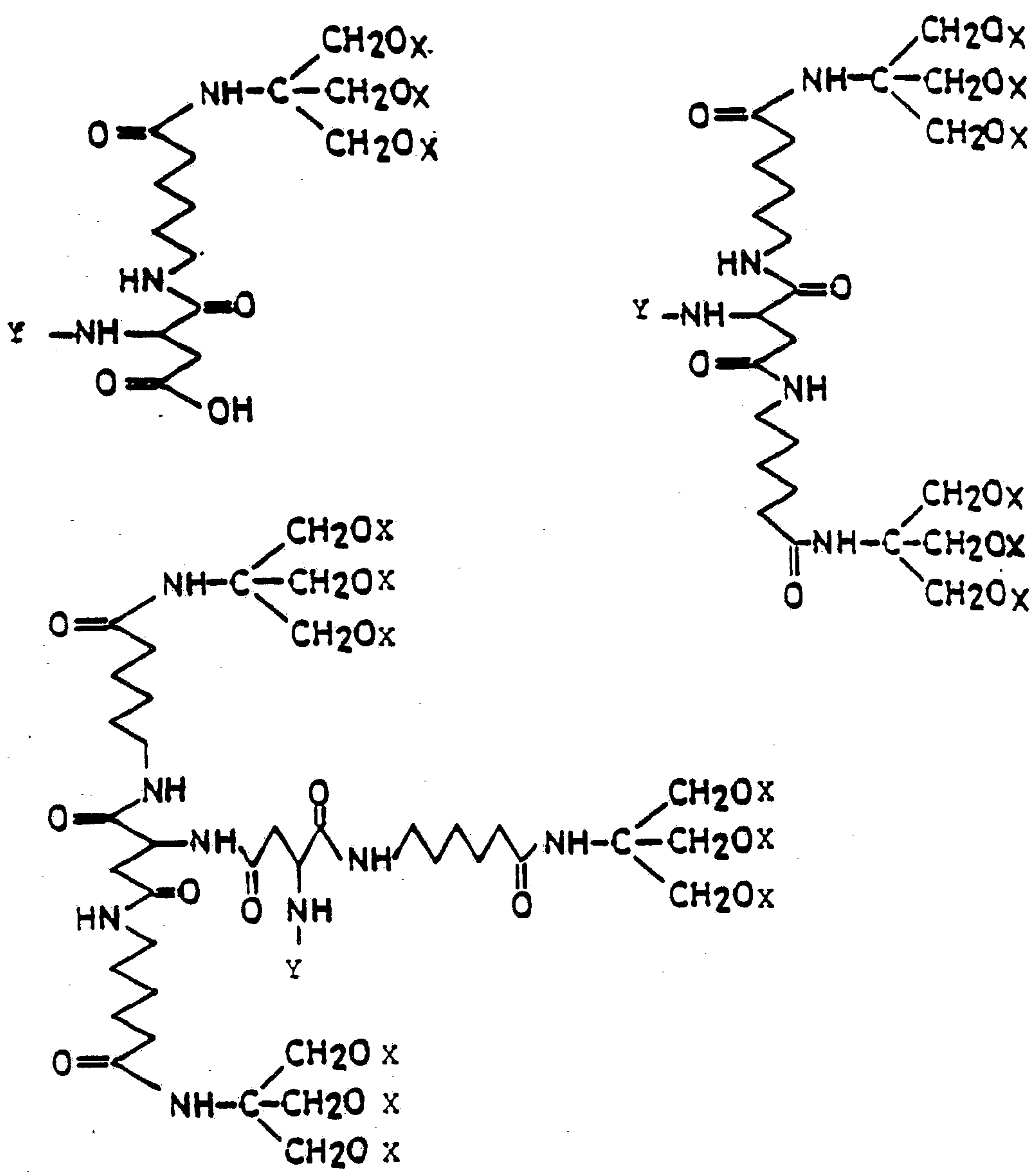

avec X identique ou différent = H ou un reste de cycle(s) glycosidique(s) de saccharide dont les fonctions hydroxy sont éventuellement protégées et

Y = H ou un groupe protecteur de la fonction amine tel que le groupe Cbz.

On peut intercaler entre O et X un reste hydrocarboné divalent notamment $-CH_2-CH_2-O-$ comme on l'a vu précédemment.

Dans ces formules, la structure chimique de base est donc constituée par l'acide aspartique ou un peptide de 2 acides aspartiques sur lequel se trouve(nt) fixé(s) un, deux ou trois restes divalents, provenant de l'acide 6-amino-hexanoïque à l'extrémité du ou desquels sont couplés des composés Tris substitués par

des cycles de saccharides à leurs extrémités.

Les restes de cycle(s) glycosidique(s) de saccharides, liés en leur position 1 sur les groupes Tris sous forme de fonctions éther, sont choisis parmi les restes suivants

β-D-galactose

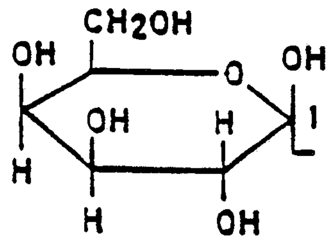

β-D-N-acetyl galactosamine

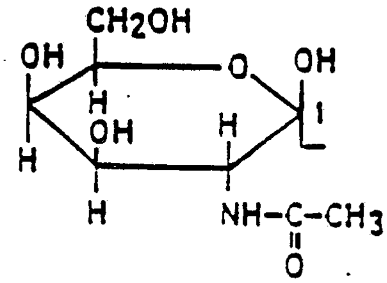

α-D-Mannose

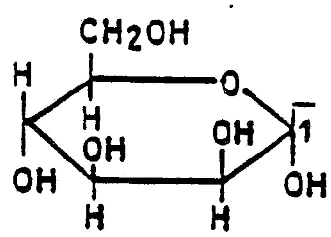

. Lactose (4-0-β-D-galactopyranosyl-D-glucose)

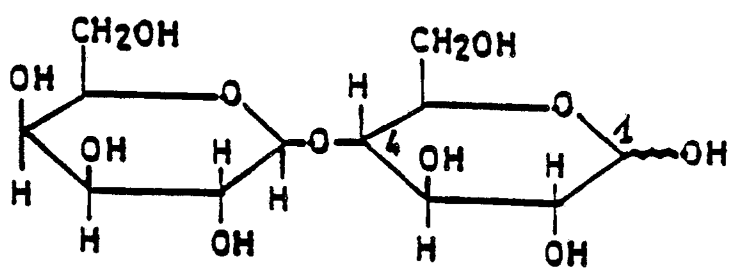

( ~ OH signifie que les deux positions alpha et beta sont possibles)

Comme on l'a vu précédemment, les β-D-galactose, N-acétylgalactosamine et lactose seront appropriés pour des vecteurs hépatotropes et les mannoses seront appropriées pour des vecteurs spécifiques du système réticulo-endothélial et les macrophages.

On peut citer plus particulièrement, comme vecteurs selon l'invention, les composés chimiques répondant aux formules suivants :

Bi-antenne

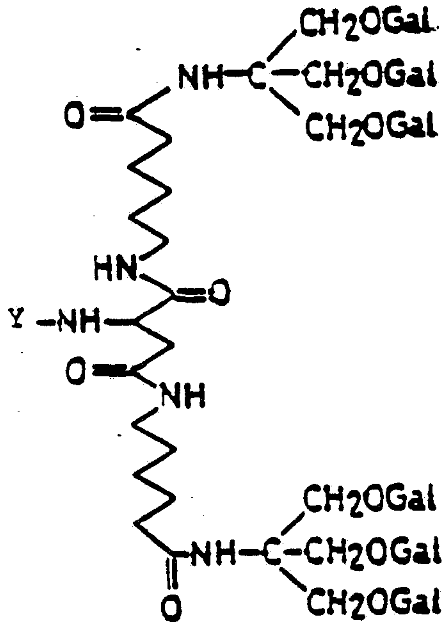

avec Gal = reste de β-D-galactose

Tri-antenne

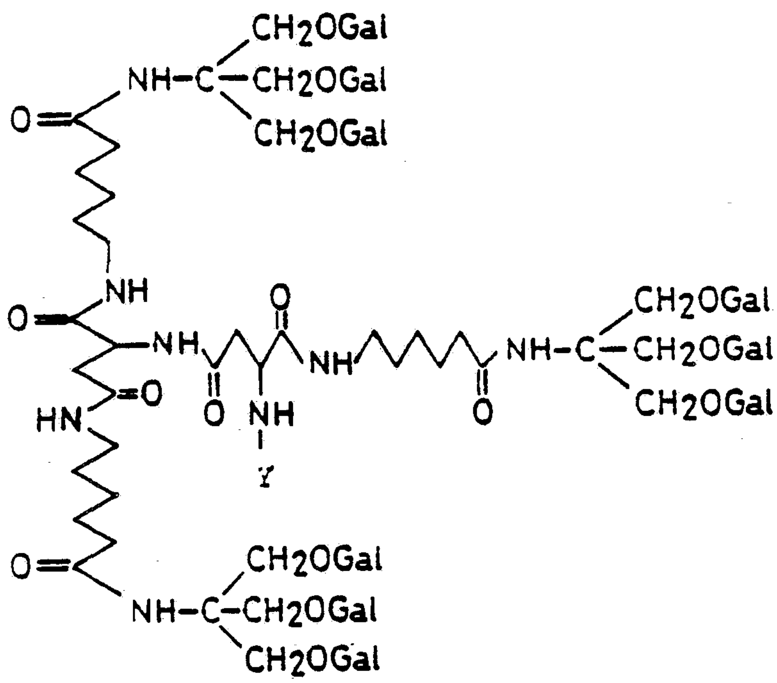

Y et Gal ayant les significations données précédemment.

Ces vecteurs sont donc particulièrement appropriés comme vecteurs hépatotropes.

Les vecteurs selon l'invention peuvent être préparés par des procédés qui seront explicités ci-après et mettent en oeuvre des réactions connues de l'homme de l'art.

De multiples approches sont possibles pour préparer les vecteurs selon l'invention.

On obtient des vecteurs selon l'invention en fixant des cycles glycosidiques sur des structures chimiques hydrocarbonées de base.

Notamment, on peut partir d'un premier composé consistant en un groupe aminoalcanoïque dont la fonction amine est éventuellement protégée et dont la fonction carboxyle est liée à l'amine d'un groupe Tris, ce dernier ayant certaines de ses fonctions hydroxy étherifiées par des restes de cycles glycosydiques de saccharides déshydroxylés en position 1, cycles dont les fonctions hydroxy sont protégées notamment par des groupes acétyle, on peut alors procéder comme suit :

1. on restaure le cas échéant la fonction amine dudit groupe aminoalcanoïque dudit premier composé par hydrogénation catalytique,

2. que l'on fait réagir avec une fonction carboxyle libre d'un acide aminé ou d'un peptide de 2 à 7 acides aminés identiques ou différents, acide aminé ou peptide ayant au moins deux fonctions carboxyle libres, et une fonction amine protégée,

3. pour obtenir un second composé qui après déprotection des cycles glycosidiques, notamment déacétylation, fournit un vecteur mono-antenne selon l'invention.

On peut obtenir un vecteur bi- ou multi-antenne à partir d'un dit second composé (mono-antenne protégée) selon qu'on le couple une ou plusieurs fois c'est-à-dire avec une ou des nouvelle(s) molécule(s) identique(s) ou différente(s) d'un dit premier composé par un lien amide entre une ou des fonction(s) carboxylique(s) restée(s) libre(s) de l'acide aminé ou du peptide dudit second composé et la ou les fonction(s) amine du ou desdits premier(s) composé(s).

On obtient alors en effet un troisième composé qui une fois déprotégé sur ses cycles glycosidiques, notamment déacétylé, donne un vecteur bi- ou multi-antenne conforme à l'invention.

On peut également préparer des vecteurs multi-antenne selon l'invention en couplant une ou plusieurs fois c'est-à-dire avec un ou plusieurs nouveaux dit(s) troisième(s) composé(s) identique(s) ou différent(s) par un lien amide entre une ou des fonction(s) carboxy libre(s) d'un dit second composé et la ou les fonction(s) amine libre(s) du ou desdits troisième(s) composé(s).

La présente invention a également pour objet des conjugués du type vecteur-médicament ou vecteur-bras-médicament, caractérisés en ce que le vecteur est conforme à l'invention. On peut citer notamment des conjugués dans lesquels le médicament est choisi parmi les substances antitumorales, antivirales et antiparasitaires. Le bras en cause consiste en un reste chimique divalent stable dans le plasma et hydrolysable dans les lysosomes. Un tel bras est nécessaire lorsque la liaison directe entre le vecteur et le médicament est, soit instable dans le plasma, soit irréversible même dans les lysosomes.

Lors de l'examen de l'activité thérapeutique des différents dérivés amino-acides de la primaquine dans le modèle de la malaria murine, le dérivé L-glutamyl-Pq a été sélectionné par son meilleur index thérapeutique.

C'est pourquoi son pilotage, vers le foie via les structures glycaniques synthétisées selon l'invention, est proposé

La présence d'une fonction carboxylique libre dans la L-glu-Pq permet de lier celle-ci aux mono, bi et tri-antennes synthétique via une fonction amine du vecteur, soit

- Pq-Glu-6-amino HexTristriGal,
- Pq-Glu Aspbis(6-amino HexTristriGal),
- Pq-Glu- Asp-Asptri(6-amino HexTristriGal).

Dans la série des dérivés amino-acides de la primaquine, la L-lysyl-Pq a également retenu l'attention. En effet, elle possède la meilleure activité, mais présente également une toxicité plus élevée que celle de la primaquine. L'adjonction de structures glycaniques sur ce dérivé diminue la toxicité de la L-lysyl-Pq tout en favorisant son accumulation sélective dans les hépatocytes.

Enfin, la présente invention a pour objet l'utilisation des vecteurs selon l'invention comme agents d'imagerie médical lorsqu'ils comportent un élément de marquage détectable, par exemple une molécule radioactive.

D'autres caractéristiques et avantages de la présente invention apparaîtront à la lumière des exemples qui vont suivre.

- La figure 1 : Expériences de compétition entre l'HSA gal marqué à l'iode-125 et différentes concentrations molaires en HSA gal non radioactives (●) ou en composés glycosylés (mono O bi ▫ triantenne ▴ ) non radioactifs. Les cellules mises en culture depuis 16 h. sont incubées à 4° C pendant 4 h. en présence de 1 ml de milieu contenant 50 ng d'$^{125}$I HSA gal et les différentes concentrations en produits non radioactifs. Après l'incubation, les cellules sont lavées et la radioactivité qui leur est associée, mesurée. Les résultats sont exprimés en pourcentage d'$^{125}$I HSA gal qui reste associé aux cellules en fonction du logarithme de la concentration molaire en compétiteur.

- La figure 2 : Expériences de compétiton entre l'HSA gal marqué à l'iode-125 et différentes concentrations molaires en galactoses d'HSA gal ou de composés glycosylés (mono O bi ▫ et triantenne ▴ )non radioactifs. Les cellules sont traitées comme décrit dans la figure 1 et les résultats sont exprimés en pourcentage d'$^{225}$I HSA gal qui reste associé aux cellules en fonction du logarithme de la concentration molaire en galactose du compétiteur.

- La figure 3 : Distribution tissulaire de la triantenne marquée au tritium. Différentes concentrations en

triantenne $^3H$ sont administrées par voie intraveineuse à des souris $OF_1$. Trois minutes après l'injection, les animaux sont sacrifiés. Le sang et les différentes organes sont prélevés, homogénéisés et la radioactivité qui leur est associée, mesurée.

Les résultats sont exprimés en pourcentage de la dose totale injectée et représentent la moyenne + déviation standard de 3 expériences.

- La figure 4 : Distribution tissulaire de la triantenne marquée au tritium. Des souris ont reçu par voie i.v. différentes concentrations de triantenne $^3H$. Les animaux sont sacrifiés 3 min. après l'injection. Le sang et les différents organes sont prélevés, homogénéisés et la radioactivité qui leur est associée, mesurée.

Les résultats sont présentés en pourcentage de la dose injectée par gr. de tissu et représentent la moyenne + déviation standard de 3 expériences.

- La figure 5 : Capture hépatique de la triantenne marquée au tritium. Différentes concentrations en triantenne $^3H$ sont injectées par voie i.v. à des souris $OF_1$. La radioactivité associée au foie est mesurée 3 min. après l'injection. Les valeurs sont exprimées en ng de galactose associés au foie en fonction des ng de galactoses injectés.

- La figure 6 : Distribution tissulaire de la triantenne marquée au tritium (a.) ou au technetium (b.).

Une concentration de 100 µg en galactoses de triantenne radioactive est injectée par voie i.v. à des souris $OF_1$. Les animaux sont sacrifiés 3 min. après l'injection. Le sang et les différents organes sont prélevés, homogénéisés et la radioactivité qui leur est associée, mesurée.

Les résultats sont exprimés en pourcentage de la dose totale administrée (case supérieure) ou en pourcentage de la dose totale injectée par gr. de tissu (case inférieure). Ils représentent la moyenne + déviation standard de 3 expériences.

Dans la description qui va suivre,

le vecteur mono-antenne avec 3 galactoses terminaux est abrégé par Cbz-Asp-(6-amino Hex-Tris tri Gal) avec 6-amino Hex = reste divalent de l'acide 6-amino hexanoïque,

le vecteur bi-antenné avec 6 galactoses terminaux par Cbz-Asp bis(-6-amino Hex-Tris tri Gal),

le vecteur tri-antenne avec 9 galactoses terminaux par Cbz-Asp-Asp-tri(-6-amino Hex-Tris tri Gal).

## EXEMPLE I

### Synthèse du N-[tri[tétra-2,3,4, 6-O-acétyl-β-D galactopyranosyloxy méthyl]méthyl] benzyloxycarboxamido-6-hexanamide (Cbz-6-amino Hex Tris tri (gal $Ac_4$))

#### I-A) Description générale

La synthèse de la structure glycanique de base (Cbz-6-amino Hex Tris tri (gal $Ac_4$)) est réalisée en adaptant le procédé décrit par KEMPEN et al. (1984), pour la synthèse du N-[[tri -D-galactopyranosyloxy méthyl] methyl] glycine.

Elle débute par la protection, via un groupement carbobenzyloxy de la fonction amine située en position 6 sur l'acide hexanoïque (M.A. BODANSKY, 1984).

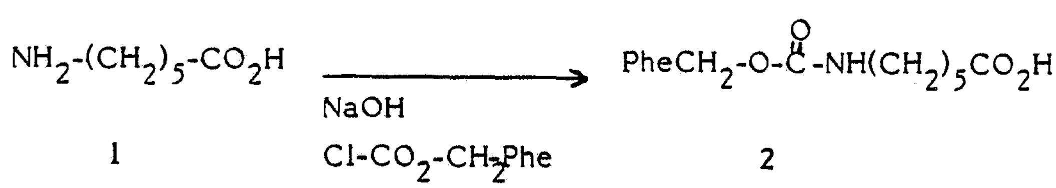


1 2

La condensation en présence de N-(éthoxycarbonyl)-2-éthoxy-1,2,dihydroquinoline (E.E.D.Q.) entre **2** et une molécule de 2-amino-2-(hydroxyméthyl)-1,3-propanediol (Tris), génère l'amide **3**.

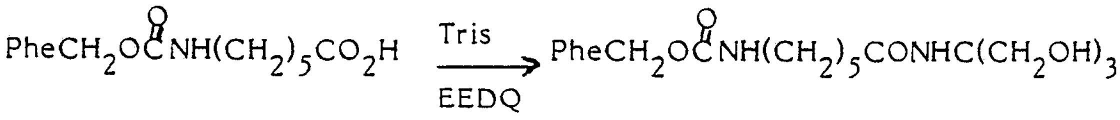


2 3

Le schéma réactionnel se poursuit par la glycosylation du composé **3** qui s'effectue en 2 étapes.

1. Synthèse du bromure de tétraacétylgalactosyl **5**
2. Réaction entre **3** et **5** pour donner **6**.

1. Synthèse du bromure de tétraacétylgalactosyl

Elle résulte de l'activation en milieu anhydride acétique, du pentaacétyl galactose **4** par du tribromure de phosphore (KEMPEN et al., 1984).

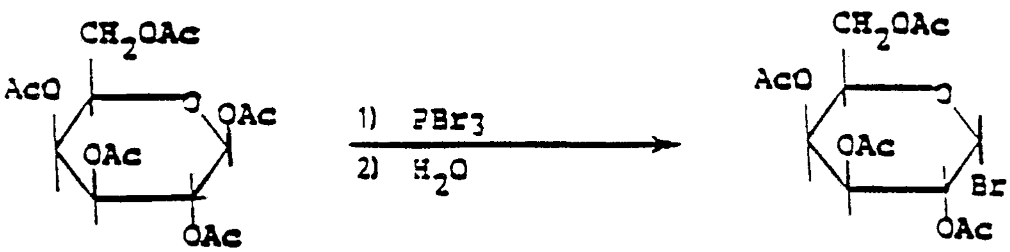


4 5

Des essais de purification ultérieure du produit bromé induisent une dégradation. Le dérivé **5** est donc directement engagé dans la réaction de glycosylation.

2. Réaction entre **3** et **5** pour donner **6**.

La réaction de glycosylation de l'amide 3 est réalisée, en milieu organique ($CH_3CN$), en présence de sels de mercure

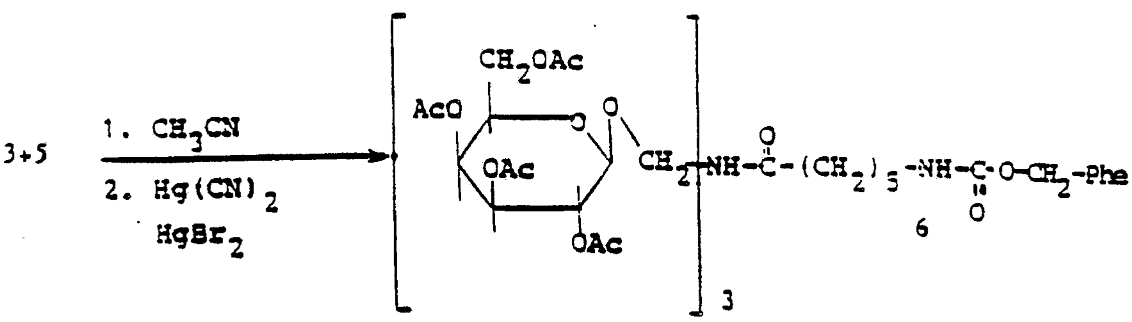


6

Le produit brut de la réaction de glycosylation est purifié par chromatographie liquide à haute pression sur gel de silice $RSilC_{18}$ (ALLTECH). Le composé **6**, est obtenu avec une pureté supérieure à 95 % sur base du chromatogramme à la longueur d'onde de 210 nm et le rendement de la réaction est de 23 %. L'analyse par résonnance magnétique nucléaire (RMN) du proton et du carbone confirme, pour le dérivé **6**, la structure attendue.

I-B) Partie expérimentale

Synthèse du N-(tri(tétra-o-acétyl-2, 3, 4, 6 -β-D galactopyranolsyloxymethyl)methyl) benzyloxycarboxamido-6-hexanamide.

1. Synthèse de l'acide benzyloxycarboxamido-6-hexanoïque

A 26.2 g (0.2 moles) d'acide amino-6-hexanoïque (Janssen 10.330.48) dissous dans 60 ml d'eau sont ajoutés 40 ml de NaOH 5N. La température du milieu réactionnel est amenée puis maintenue à 10 °C grâce à un bain eau-glace. Après stabilisation de la température, le benzylchlorocarbonate (37.4 g, 31.6 ml, 0.22 moles - janssen 15.294.65) et le NaOH 2 N (110 ml) sont ajoutés tour à tour et sous agitation vigoureuse.

Le temps total d'addition est d'une heure et demie.

La réaction se poursuit pendant une demi-heure supplémentaire. Le pH de la solution est alors ajusté à 10.

La solution est ensuite extraite à l'éther (4 x 100 ml). La phase aqueuse est acidifiée à pH-1 par de l'HCl 5 N (+/- 40 ml). La phase huileuse qui en résulte est à nouveau extraite à l'éther (4 x 80 ml). Les différentes phases organiques sont rassemblées, séchées, filtrées et évaporées.

les 52 g de poudre blanche obtenus sont conservés à température ambiante, sous atmosphère d'argon. Le produit est homogène en CCM dans le système A, révélation à l'iode. (Rf = 0,58).

Rdt : 89 %

pF : 63 °C

RMN$^1$H (200 MHz - $CDCl_3$ - TMS)

(ppm) : 1.3 à 1.74 (massif, 6H) ; 2.34 (t,2H) ; 3.18 (m,2H)₃ 4.8 (massif, 1H) ; 5.09 (s,2H) ; 7.34 (s,5H).

2. Synthèse du N-(tris(hydroxymethyl)méthyl)benzyloxycarboxamido-6-hexanamide

A 9.1 g (75 mmoles) de Tris (p.A. Janssen 16.762.78) dissous dans 750 ml d'éthanol absolu sont ajoutés 22 g (82.5 mmoles) d'acide 6-amino-hexanoique protégé et 22.2 g (90 mmoles) de N-(ethoxy-carbonyl)ethoxy-2 dihydro-1,2-quinoline (EEDQ - Janssen 16.535.45). La solution est chauffée à reflux pendant 6 heures puis refroidie à température ambiante avant d'être évaporée. L'huile obtenue cristallise dans 375 ml de diethylether en une nuit. Les cristaux blancs sont filtrés et recristallisés à chaud dans 375 ml d'acétate d'éthyle.

15.4 g (42 mmole) de N-(tris(hydroxyméthyl)méthyl)benzyloxycarboxamido-6-hexanamide sont récupérés. Le produit est homogène en CCM dans le système B-révélation à l'iode (Rf = 0.36). Il est conservé sous atmosphère d'argon à - 20 °C.

Rdt : 56

Point de fusion 88-90 °C

RMN$^1$H (200 MHz - $CDCl_3$ - TMS)

(ppm) : 1.48 (m,2H) ; 1.51 (m,2H) ; 1.64 (m,2H) ; 2.24 (t,2H) ; 3.17 (9,24) ; 3.59 et 3.62 (s,64) ; 4.64 (m,3H) ; 49 (massif, 1H) ; 5.08 (s,2H) 6.57 (s,1H) ; 7.34 (s,5H)

I.R. (KBr)

($cm^{-1}$) : 3300 (broad), 2940, 2860, 1680, 1615, 1550, 1520, 1460, 1270, 1130, 1040, 1020

3. Synthèse du bromure de tétra-O-acétyl-2, 3, 4, 6 - -D-galactopyranosyle

15 g (38.4 mmoles) de penta-O-acetyl-beta-D-galactopyranose (Janssen 15.861.50) sont dissous dans 76 ml d'anhydride acétique (Sigma A. 6404). La solution est refroidie et maintenue entre -5 et 10 ° grâce à un bain eau/glace/NaCl.

A froid, et sous agitation vigoureuse, sont ajoutés 23 ml de tribromure de phosphore ($PBr_3$ Janssen 16.947.69), puis goutte à goutte, 33 ml d'eau. Après 5 heures, temps total d'addition, 270 ml de chloroforme refroidi à 4 °C sont introduits dans le ballon.

La phase chloroforme est lavée par de l'eau à 4 °C (4 x 300 ml) puis par une solution à 4 °C de bicarbonate de sodium à 7 % dans l'eau (2 x 300 ml). La phase organique est séchée, filtrée puis évaporée. L'adjonction d'hexane fournit un précipité de 12.6 g de bromure de tétra-O-acétyl 2, 3, 4, 6 -D-galactopyranosyle.

La réaction est suivie par C.C.M. dans le système C -révélation à l'orcinol. La chromatographie montre la disparition totale du produit de départ (Rf départ = 0.24 ; Rf bromé = 0.36).

Rdt : 80 %

pF : 77 - 80°C

RMN$^1$H (MHz ; $CDCl_3$ - TMS)

(ppm) : 2.02 (s,3H) ; 2.06 (s,3H) ; 2.12 (s,3H) ; 2.16 (s,3H) ; 4.15 (m,2H) ; 4.49 (dt, 1H, $j_{5-6}$ = 6.1 Hz, $j_{5-6}$ = 08 Hz) ; 5.05 (dd, 1H, $J_{2-1}$ = 3.96 hz, $J_{2-3}$ = 10.6 Hz) ; 5.41 (dd, 1H, $j_{3-4}$ = 3.36 Hz, $J_{2-3}$ = 10.55 Hz) ; 5.52 (dd, 1H, $J_{4-5}$ = 0.98 Hz, $j_{4-3}$ = 3.18 Hz) ; 6.70 (d, 1H, $j_{1-2}$ = 3.91 Hz).

4. Synthèse du N (tri(tétra-o-acétyl-2, 3, 4, 6-beta-D-galactopyranosyl-oxyméthyl)méthyl) benzyloxycarbonamido-6-hexanamide ou Cbz-6-amino Hex Tris tri(galAc$_4$)

a) synthèse

Un ballon de 500 ml à trois cols est équipé d'un robinet, d'un bouchon et d'une ampoule à addition surmontée d'une trappe contenant de l'acide sulfurique concentré.

Après avoir purgé le montage à l'argon, 2.8 g (7.62 mmoles) de dérivé N-(tri(hydroxymethyl)methyl)-hexanamide sont placés dans le ballon réactionnel suivi de 64 ml d'acétonitrile fraîchement distillé sur hydrure de calcium ($CaH_2$, 0-10 mm de taille de grains - Janssen 21.332.2).

3.8 g (15.31 mmoles) de cyanure de mercure ($Hg(CN)_2$ p.a. Janssen 19.690.96) et 5.6 g (15.31 mmoles) de bromure de mercure ($HgBr_2$ p.a. Janssen 19.040.37) sont ensuite ajoutés, sous atmosphère d'argon, au mélange réactionnel. Après dissolution de sels de mercure, 64 ml d'une solution d'acétonitrile fraîchement distillée et contenant 12.6 g (30.67 mmoles) de bromure de tétra-o-acétyl galactopyranosyle sont introduits dans l'ampoule et additionnés goutte à goutte au mélange réactionnel. Lorsque l'addition est terminée, la réaction se poursuit sous agitation magnétique et à l'abri de la lumière pendant 16 heures.

A la fin de la réaction, la solution est évaporée sous vide. Le résidu est redissous dans 180 ml de chloroforme. La phase organique est lavée 5 fois avec 100 ml d'une solution 1 M de KBr puis 5 fois avec 90 ml d'eau.

Après les lavages, la phase $CHCl_3$ est séchée, filtrée et évaporée pour donner 12 g de résidu blanchâtre.

Une CCM dans le système D-révélation à l'orcinol, montre un mélange de 6 produits dont un majeur.

b) purification

Elle est réalisée par CLHP, 6 g de produit brut sont dissous dans un mélange acétonitrile /THF/$H_2O$ en rapport 32/11/57. Le volume final à injecter est d'environ 250 ml. Il est filtré sur membrane Millipore HVLP avant d'être injecté par pompage direct au travers de la pompe grâce à la présence d'une vanne "2 voies" située entre l'arrivée des solvants et le corps de pompe. Le débit pendant l'injection est de 16 ml/mn.

Lorsque toute la solution à injecter est passée en tête de colonne, le pompage des solvants est rétabli par la vanne "2 voies" et sa composition en $CH_3CN$/THF/$H_2O$ est respectivement de 30/10/60. Le débit est porté à 20 ml/min.

Après 30 min, la composition $CH_3CN$/THF/$H_2O$ passe à 32/12/56.

Après 95 min, des fractions de 17 ml sont collectées à la sortie du détecteur UV par l'intermédiaire d'un collecteur programmable LKB.

Le dérivé Cbz-6-amino Hex Tris tri(galAc$_4$) élue vers 120-130 min.

Après environ 200 min, les proportions d'acétonitrile et de THF sont augmentées de 1 % chacune.

Après environ 250 min, la colonne est lavée avec un mélange $CH_3CN$/THF/$H_2O$ en proportion 35/15/50.

Les différentes fractions sont contrôlées par C.C.M. ou CLHP système analytique dont la partie aqueuse de la phase mobile contient 0.05 % de TFA.

c) analyses

. chromatographie liquide à haute performance

Dans les conditions CLHP analytique sur colonne $C_{18}$ dont la phase mobile est constituée du mélange (50/50, v/v) $H_2O$ 0.05 % TFA/$CH_3CN$, les différents constituants du mélange brut sont effectivement séparés et le $T_R$ du produit majeur est de 119 +/- 01 min.

Après purification, 1.2 g de poudre blanchâtre sont obtenus (rdt : 23 %) avec une pureté supérieure à 95 %, sur base du profil obtenu à la longueur d'onde de 210 nm.

. résonance magnétique nucléaire du proton et du carbone 13

RMN$^1$H ($CDCl_3$TMS)
(ppm) : 1.2 à 1.45 (massif, 2H) ; 1.45 à 1.7 (massif, 4H) ; 1.98 (s,9H) ; 2.06 (s, 9H) ; 2.07 (s,9H) ; 2.15 (s,9H) 3.2 (9,2H) ; 3.76 (d,1H,$J_{H-N}$ = 10.07 hz) ; 3.92 (t,1H,$J_{H-N}$ = 6.84 Hz) ; 4.12 (m,9H) ; 4.42 (d,1H, $J_{H-N}$ = 7,46 Hz) ; 5.06 (m,8H) ; 5.39 (d,1H, $J_{H-N}$ = 2.44 Hz) ; 5.91 (s, 1H) ; 7.35 (s,5H).
RMN$^{13}$C ($CDCl_3$-TMS)
(ppm) : 19.2, 24.4, 25.4, 28.8, 36.2, 40.4, 58.4, 62, 66, 66.4, 68, 68.6, 70, 70.2, 103, 128.2, 126.6, 156.8, 169.8, 170.4, 170.6, 170.8, 173.6

## EXEMPLE 2

### Synthèse de structures glycaniques complexes (mono, bi, tri-antennes)

I - Description générale

A partir de l'unité structurale **6**, on élabore des structures plus complexes, caractérisées par la présence d'un nombre croissant de galactoses (3, 6, 9).

Ces différentes synthèses sont effectuées au départ de :
- l'unité **6** complètement déprotégée, c'est-à-dire dont la fonction amine de la partie hexanoïque ainsi que les 4 groupements hydroxyles des galactoses sont restaurés (voie des galactoses déprotégés).
- l'unité **6** partiellement déprotégée, c'est-à-dire dont seule la fonction amine de la partie hexanoïque est restaurée (voie des galactoses protégés).

Les deux voies de synthèse impliquent des réactions de condensation entre, d'une part, la fonction amine libre du composé glycosylé et, d'autre part, les deux fonctions carboxyliques de l'acide aspartique. Elles sont illustrées par les schémas 1 et 2.

A. Voie des galactoses déprotégés

La déprotection complète de l'unité glycosidique **6** est effectuée en deux temps (KEMPEN et al., 1984).
- une réaction de déacétylation en milieu triéthylamine-méthanol-eau, permet de restituer les groupements hydroxyles des galactoses.
- une réaction d'hydrogénation catalytique (Pd/C) en milieu acide acétique (60 %) restaure la fonction amine de la partie hexanoïque.

Le composé **7** représente l'unité glycosidique complètement déprotégée.

Deux approches sont ensuite envisagées pour coupler **7** sur les fonctions carboxyliques de l'acide aspartique.

Dans la première approche, c'est l'EEDQ qui sert d'agent de condensation (LEE et al., 1983).

La seconde utilise l'acide aspartique activé sous forme de N-hydroxysuccinimidyl ester par l'action de la dicyclohexylcarbodiimide (DCC) en présence de NHS (N-hydroxy-succinimide) (BODANSKY, 1984).

Quelle que soit l'approche envisagée pour coupler l'unité glycosidique déprotégée **7** sur les fonctions carboxyliques de l'acide aspartique, il n'a pas pu être mis en évidence un composé dont la structure correspondrait à une bi-antenne (deux fonctions carboxyliques substituées). Par contre, les conditions utilisées favorisent la synthèse de la mono-antenne (une des deux fonctions carboxyliques substituées). La purification par filtration moléculaire sur biogel du produit brut de la réaction aboutit en effet, quel que soit

l'agent de couplage utilisé au même composé **8** ou mono-antenne (schéma 1 ci-après).

Et l'analyse par RMN établit que la substitution s'effectue préférentiellement sur la fonction carboxylique située en alpha (**8b**). On a également constaté, dans cette première voie de synthèse, que le couplage via l'ester activé (DCC,NHS) améliore de façon significative la synthèse de la mono-antenne. En effet, le produit brut de la réaction est constitué de trois composés au lieu des cinq obtenus lors de l'utilisation de l'EEDQ comme agent de condensation.

Une des raisons pour lesquelles la synthèse d'une biantenne ne se serait pas ou très mal effectuée, pourrait provenir de l'obligation d'utiliser une phase aqueuse pour coupler l'unité glycosidique déprotégée, sur l'acide aspartique.

C'est pourquoi, nous nous sommes tournés vers une autre voie de synthèse dans laquelle la réaction de glycosylation pourrait s'effectuer en milieu organique et de façon séquentielle. Ce type d'approche aurait l'avantage de permettre la purification et la caractérisation des produits étape par étape.

B. Voie des galactoses protégés

La synthèse des mono, bi et tri-antennes est réalisée à partir de l'unité glycosidique dont seule la fonction amine de la partie hexanoïque est restaurée.

## B.1. Synthèse monoantenne

L'unité structurale **6** est soumise à une hydrogénation catalytique (Pd/C) en milieu acide formique (85 %) pour donner le composé **10** (Paulsen P. et al, 1986).

La restauration de la fonction amine permet au dérivé de réagir avec l'anhydride aspartique formé à partir d'acide aspartique et d'anhydride acétique (Chim. Ping Yong, 1986).

La structure du produit d'ouverture de l'anhydride acétique correspond au composé **11** ou mono-antenne protégée (analyse par RMN).

Soumis à une réaction de déacétylation puis à une filtration moléculaire sur biogel, le produit **11** est transformé en dérivé **8** ou mono-antenne.

Cette première étape permet donc la synthèse d'une monoantenne dont la structure correspond à celle obtenue par la première voie de synthèse via les galactoses déprotégés.

## B.2. Synthèse de la biantenne

Dans ce cas-ci aussi, deux approches sont envisagées pour coupler une nouvelle molécule de **10** sur la fonction carboxylique restée libre de **11**.

Dans la première, le produit d'ouverture de l'anhydride aspartique est activé à l'EEDQ.

La deuxième utilise la mono-antenne activée à la DCC en présence de NHS.

Dans les deux approches, le produit activé est mis en réaction avec 1.2 équivalents de dérivé **10**.

La purification des produits bruts de réaction par chromatographie liquide à haute pression sur gel de silice ($RSiIC_{18}$) permet d'obtenir la bi-antenne protégée ou composé **12** avec une pureté supérieure à 90 %.

La structure de **12**, ainsi synthétisé, est confirmée par l'analyse RMN.

L'utilisation de l'ester activé à la DCC en présence de NHS semble dans ce cas-ci aussi être préférable à une activation à l'EEDQ. L'analyse par RMN du produit brut de la réaction via l'EEDQ montre en effet une réaction secondaire entre l'EEDQ et le composé **10**.

Une réaction de déacétylation en milieu TEA-MEOH-$H_2O$ suivie d'une filtration moléculaire sur biogel de **12** permet d'obtenir le composé **9** ou bi-antenne.

## B.3 Synthese de la tri-antenne (Schéma 2 ci-après)

La synthèse qui mène au composé triantenne **14** est le résultat de la combinaison d'une mono-antenne

et d'une bi-antenne.

Pour y accéder, le composé **12** subit une hydrogénation catalytique (Pd/C) en milieu acide formique et donne le dérivé **13**.

Ce dernier est ensuite mis en réaction avec le composé **11** activé à la DCC en présence de NHS.

La purification par chromatographie à haute pression sur gel de silice $C_{18}$ permet l'obtention de la tri-antenne protégée (composé **14**) dont la pureté est supérieure à 90 %.

Une réaction de déacétylation en milieu TEA-MEOH-$H_2O$, suivie d'une purification sur biogel de **14**, fournit le composé **15** ou tri-antenne.

**Schéma 1.** Voies de synthèse de la mono et de la bi-antenne

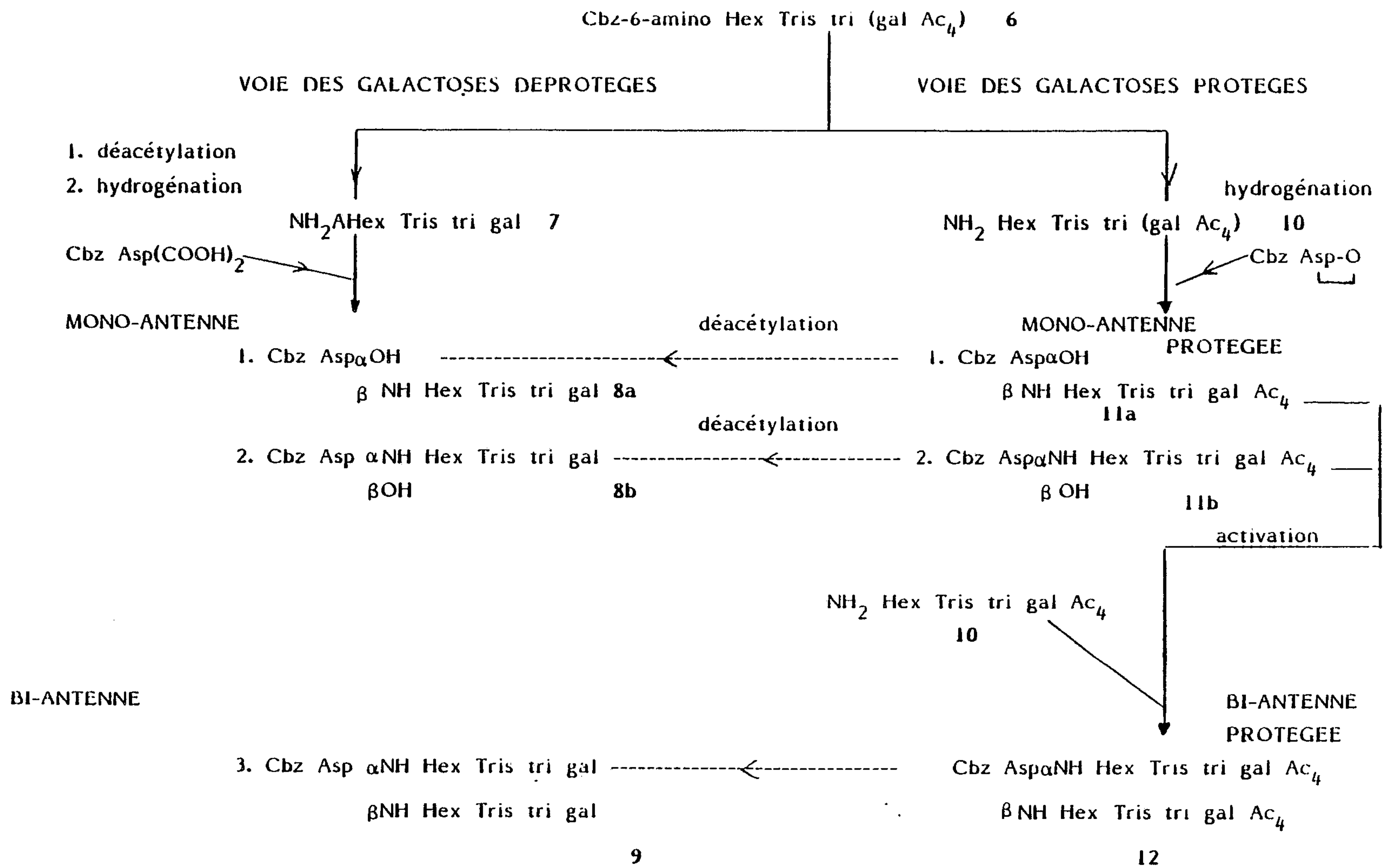

Schéma 2 : Synthèse de la triantenne

Cbz Asp α NH Hex Tris tri (gal $Ac_4$)
β NH Hex Tris tri (gal $Ac_4$)

12

hydrogénation

$NH_2$ Asp α NH Hex Tris tri (gal $Ac_4$)
β NH Hex Tris tri (gal $Ac_4$)

13

Cbz Asp α NH Hex Tri tri (gal $Ac_4$)
β OH

11b

activation

Cbz Asp NH Hex Tris tri (gal $Ac_4$)
NH Asp α NH Hex Tris tri (gal $Ac_4$)
β NH Hex Tris tri (gal $Ac_4$)

14

1. Déacétylation
2. Filtration moléculaire sur biogel

Cbz Asp NH Hex Tris tri gal
β NH Asp α NH Hex Tris tri gal
β NH Hex Tris tri gal

15

## II - Partie expérimentale

### 1. Synthèse selon la voie "galactoses déprotégés"

#### 1.1 Réaction de déacétylation des galactoses

8.67 ml d'eau et 1.146 ml (8.22 mmoles) de triéthylamine (Merck 808352) distillée sur KOH et conservée sous atmosphère d'argon, sont ajoutés goutte à goutte à 930 mg (0.685 mmoles de Cbz-6-amino Hex Tris tri (gal $Ac_4$) dissous dans 8.67 ml de méthanol (carlo ERBA).

L'addition s'effectue sous agitation et à température ambiante.

L'évolution de la réaction est suivie par CCM dans le système A - révélation à l'orcinol.

Après 2 heures, la réaction est complère. La solution est acidifiée en ajoutant 0.468 ml (8.22 mmoles) d'acide acétique glacial.

Après 15 min, le mélange est évaporé. le résidu sec obtenu est redissous dans 100-150 ml d'eau et lyophilisé. La présence de 2 impuretés dans le produit brut de la réaction est mise en évidence par CCM dans ler système A. Ces impuretés sont éliminées par chromatographie à pression moyenne. Pour ce faire, le composé lyophilisé est dissous dans 7 ml de mélange dichlorométhane-méthanol-eau (10/1/1, v/v) puis déposé sur une colonne contenant le gel de silice lavé par le mélange (10/1/1/) cité plus haut.

Les fractions collectées sont analysées par CCM dans le solvant A. Celles qui contiennent le produit majeur sont rassemblées et la solution qui en résulte évaporée.

Le résidu sec obtenu est remis en solution dans 100-150 ml d'eau et la solution lyophilisée.

Après lyophilisation, 400 mg de dérivés déacetylé sont récupérés.

Rdt : 68 %

#### 1.2 Hydrogénolyse du groupement benzyloxycarbonyle

350 mg (0.410 mmoles) du dérivé déacétylé sont hydrogénés dans 45 ml d'une solution acide acétique, eau (60/40) en présence de 150 mg de palladium sur charbon (Janssen 19.503.06).

la solution est maintenue sous agitation et sous une pression en hydrogène de 20 psi pendant 16 h.

A la fin de la réaction, le catalyseur est éliminé par filtration sur une membrane HVLP (Millipore). Le filtrat est dilué dans 100 ml d'eau et lyophilisé.

Après 2 lyophilisations successives, 293 mg (0,407 mmoles) de dérivé déprotégé sont récupérés.

Un seul produit s'observe en C.C.M. dans le système E, révélation à l'orcinol et à la ninhydrine.

#### 1.3 Réaction de couplage entre le 6-amino Hex Tris tri gal et l'acide N-alpha-bensyloxycarbonyl aspartique

a) couplage en présence de E.E.D.Q.

270 mg (0375 mmoles) de produit déprotégé et 42 mg (0,158 mmoles) d'acide N-ALPHA-benzyloxycarbonyl aspartique (Janssen 17.205.36) sont mis en solution dans 13.5 ml d'eau. Après dissolution complète, 514 mg (208 mmoles) de E.E.D.Q. (Janssen 16.535.45) sont additionnés.

Sous agitation du mélange sont encore ajoutés, goutte à goutte, 13.5 ml d'éthanol absolu.

la présence de 5 composés dont un majeur est mise en évidence par C.C.M. dans le système E, révélation à l'orcinol. Le produit brut est purifié sur Biogel $P_4$ : 290 mg sont dissous dans 7 ml d'acide acétique puis déposés sur la colonne.

Une aliquote des différentes fractions est analysée par CCM dans le système E.

Le volume d'élution du composé majeur est de 274 ml. Deux cycles de lyophilisation permettent d'obtenir 61 mg de poudre blanchâtre. Sa pureté est de 95 %. Elle est déterminée d'après le profil chromatographique à 210 nm sur colonne carbohydrate de phase mobile constituée d'acétonitrile et d'eau (7/3-v/v). Le $T_R$ du produit est, dans ces conditions, de 16.9 +/- 01 min.

Rdt : 40 %

RMN¹H

b) réaction de couplage via l'ester activé obtenu par l'action du NHS et de la DCC sur l'acide N-alpha-benzyloxycarbonyl asparatique

37.4 mg (0.140 mmole) d'acide N-benzyloxycarbonyl aspartique et 32.2 mg (0.280 mmoles) de NHS sont introduits dans un ballon contenant 3 ml d'acétate d'éthyle distillé sur KOH.

La solution est alors refroidie et maintenue à 4° C grâce à un bain eau/glace, avant d'y ajouter 58 mg (0.880 mmoles) de DCC.

La réaction est suivie par CCM dans le système B, révélation au vert de bromocrésol (Merck art. 8121 : 40 mg sont dissous dans 100 ml d'éthanol. De la soude (0.1 M) est ensuite ajoutée à cette solution jusqu'à l'apparition d'une coloration bleue).

Après 20 heures à 4° C, 0.5 équivalents de NHS et de DCC sont rajoutés à la solution.

Après un temps total de réactionde 36 heures, la dicyclohexylurée est éliminée par filtration sur membrane FH (Millipore). Le filtrat est évaporé à sec et 77 mg (0.137 mmoles) de produit activé sont récupérés.

38 mg (68.5 mmoles) d'ester activé ainsi obtenus sont dissous dans 1 ml de $CH_3CN$ et mis en réaction avec 1.67 ml d'eau contenant 102 mg (0.140 mmoles) de 6-amino Hex Tris tri gal.

Après 24 heures de réaction à température ambiante et sous agitation constante, le solvant est évaporé. Le produit est mis en suspension dans un volume d'environ 100 ml et lyophilisé.

Après lyophilisation, 110 mg de poudre blanchâtre sont obtenus. L'analyse CCM dans les mêmes conditions que celles décrites plus haut (1.3 a)) révèle la présence de 3 composés dont le produit majoritaire présente, par CLHP (voir 1.3 a)), les mêmes caractéristiques que celui obtenu lors du couplage en présence de E.E.D.Q.

2. <u>Synthèse selon la voie "galactoses protégés"</u>

2.1 Synthèse de la monoantenne

2.1.1 Hydrogénolyse du Cbz-6-amino Hex Tris tri (gal $Ac_4$) :

3.4 g (2.5 mmoles) de Cbz-6-amino Hex Tris tri (gal $Ac_4$) sont ajoutés à 1.7 g de palladium sur charbon en suspension dans 100 ml d'un mélange acide formique-eau (85/15). La solution est maintenue sous agitation et sous une pression en hydrogène de 20 psi pendant 16 heures. En fin de réaction, le catalyseur est éliminé par filtration sur membrane HVLP et le filtrat évaporé à sec. Le résidu obtenu est redissous dans 200 ml de chloroforme. La phase organique est lavée 5 fois par 100 ml de bicarbonate ($NaHCO_3$) à 5 % (p/v) dans l'eau, séchée, filtrée et évaporée. 287 mg de poudre blanchâtre sont récupérés.
Rdt : 94 %.

2.1.2 Synthèse de l'anhydride aspartique

8 g (29.9 mmoles) d'acide N-alpha-benzyloxycarbonyl aspartique (Janssen 17.20536) sont dissous dans 8 ml d'anhydride acétique (Janssen 22. 213.97). La solution est amenée puis maintenue à 45° C pendant 2 heures. En fin de réaction, la solution est brutalement refroidie dans un bain eau-glace.

Le précipité blanc est filtré, lavé 3 fois à l'hexane puis séché sous vide. Les 7.3 g de poudre blanche sont directement engagés dans la réaction suivante.

2.1.3 Réaction entre le produit hydrogéné et l'anhydride de l'acide aspartique

Un ballon à 2 cols est équipé d'un robinet et d'un tube contenant du chlorure de calcium ($CaCl_2$). L'appareillage est purgé à l'argon avant d'y introduire 7.3 g (14.64 mmoles d'anhydride aspartique, 70 ml de chloroforme et 1 g (0.816 mmoles) de 6-amino Hex Tris tri (gal $Ac_4$). Après 24 heures à température ambiante, la solution est évaporée. Le résidu obtenu est redissous dans 200 ml de chloroforme. La phase organique est lavée 5 fois par 250 ml de $NaHCO_3$ à 5 % (p/v) dans l'eau et 5 fois par 150 ml d'acide citrique à 4 % (p/v) dans l'eau. La phase chloroforme séchée, filtrée puis évaporée fournit 1.1 g de

monoantenne protégée.
Rdt : 91 %
Masse : 1474 ($M^+$)
RMN$^1$H
$^{13}$C
RMN$^1$H ($cDcl_3$ - TMS) :
ppm 1.2 à 1.4 (massif) ; 1.48 à 1.6 (massif) ; 1,98 (s) ; 2.05 (s) ; 2.07 (s) ; 2.16 (s) ; 2.68 (m) ; 3 (m) ; 3.3 (massif) ; 3.73 (d) ; 3.98 (t élargi) ; 4.14 (m) ; 4.46 (d) ; 5.1 (m) ; 5.40 (d) ; 5.09 (s) ; 6.11 (s) ; 7.39 (s).
RMN$^{13}$C (CD $Cl_3$ - TMS) :
ppm 25 (T) ; 26 (T) ; 29.7 (T) ; 31.6 (T) ; 36.7 (T) ; 39.1 (T) ; 51.4 (D); 59.2 (S) ; 60 (D) ; 67 (D) ; 67.2 (T) ; 68.3 (T) ; 69.2 (D) ; 70.6 ; 70.8 (D) ; 101.4 (D) ; 128.16 (D) ; 128.3 (D) ; 128.6 (D) ; 136.2 (S) ; 155.9 (S) ; 169.6 (S) ; 170 (S) ; 170.2 (S) ; 170.6 (S) ; 172.9 (S) ; 174.2 (S).

2.1.4 Réaction de déacétylation

2.5 ml d'eau contenant 0.720 mmoles de TEA sont ajoutés goutte à goutte à 32 mg (0.022 mmoles) de monoantenne protégée dissous dans 2.5 ml de méthanol. Après 2 heures, la réaction est complète (CCM -système A - révélation à l'orcinol). La solutionest alors acidifiée en ajoutant 40 $\mu l$ (0720 mmoles) d'acide acétique glacial. Après 15 min, le mélange est évaporé. Le produit sec obtenu est remis en suspension dans environ 30 ml d'eau. Cette solution lyophilisée plusieurs fois donne 19 mg de poudre blanche.

Le produit est homogène dans différents systèmes de solvants (CCM dans le système E ; CCM-$NH_2$ dans le système F). Il possède les mêmes caractéristiques que le composé décrit en 2.1.3.

2.2 Synthèse de la biantenne

2.2.1 Couplage du 6 amino Hex Tris tri (gal $Ac_4$) avec la monoantenne, en présence de E.E.D.Q.

150 mg (0.102 mmoles) de monoantenne protégée sont mis en solution dans 5 ml d'acétonitrile.

250 mg (1.02 mmoles) de E.E.D.Q. et 256 mg (0.209 mmoles) de 6-amino Hex Tris tri (gal $Ac_4$) sont ensuite ajoutés.

Après 24 heures sous agitation à température ambiante, la solution est filtrée sur membrane Millipore FH puis évaporée. Le résidu obtenu est remis en solution dans 200 ml de chloroforme. La phase organique est lavée 5 fois par 100 ml d'eau, séchée, filtrée et évaporée. Une CCM dans le système G, révélation à l'orcinol, du produit brut de la réaction montre la présence de 2 produits majeurs (Rf = 0.89 ; Rf 0.51) et de un mineur (Rf = 0).

les 350 mg de produit brut sont ensuite purifiés par chromatographie à pression moyenne.

L'éluant est constitué de $CH_2Cl_2$-$CO(CH_3)_2$ en proportion 10/5. Il permet d'éluer le composé de Rf = 089. La polarité de la phase éluante est ensuite augmentée (5/10) et permet l'élution du composé de Rf = 051. Après évaporation des solvants, 185 mg et 150 mg des deux produits sont respectivement récupérés.

2.2.2. Réaction du 6 amino Hex Tris tri (gal $Ac_4$) sur l'ester activé de la monoantenne

500 mg (0339 mmoles) de monoantenne protégée et 57 mg (1.2 équivalents) de NHS sont dissous dans 14 ml d'acétate d'éthyle distillé. La solution est refroidie puis maintenue à 4° C grâce à un bain eau-glace, avant d'y ajouter 101 mg (1.2 équivalents) de DCC.

Après 20 heures à 4° C, la dicyclohexylurée formée est éliminée par filtration sur membrane FH et le filtrat est évaporé à sec.

L'entièreté de l'ester activé de la monoantenne est dissous dans 30 ml d'acétonitrile et mis en réaction avec 400 mg (0.349 mmoles) de 6-amino Hex Tris tri (gal $Ac_4$).

Après 20 heures d'agitation à température ambiante, l'acétonitrile est évaporé et le produit sec qui en résulte est redissous dans 150 ml de $CHCl_3$. La phase organique est lavée 5 fois par 150 ml d'eau, séchée, filtrée et évaporée pour donner 775 mg de résidu blanchâtre.

Le produit brut est purifié par CLHP préparatif : les 775 mg sont dissous dans 56 ml d'un mélange constitué d'eau, d'acétonitrile et de THF en proportion (56/33/11).

Après son dépôt en tête de colonne, celle-ci est éluée avec le même mélange en proportion (50/36/14) et au débit de 20 ml/min. Dans ces conditions, le $T_R$ de la biantenne protégée est de 66 min. La purification permet d'obtenir 360 mg de poudre blanchâtre.

Rdt : 46 %

RMN$^1$H

$^{13}$C

RMN$^1$H ($CDCl_3$ - TMS) :

ppm 1.2 à 1.4 (massif, 4H) ; 1.5 à 1.6 (massif, 8H) ; 2. (s, a) ; 2.07 (s, b) ; 2.1 (s, c) ; 2.17 (s, d) ; 2.2 (s, a + b + c + d = 76H) ; 2.55 (m, 2H) ; 2.8 (m, 1H) ; 3.2 (massif 4H) ; 3.8 (t, 6H) 3.9 (t élargi, 6H) ; 4.18 (m, 18 H) ; 4.5 (d, 6H) ; 5.1 (m, 14H) ; 5.4 s élargi, 6H) ; 6.0 (s, 1H) ; 6.1 (s, 1H) ; 7.35 (s, 5H).

RMN$^{13}$c : ($cDcl_3$ - TMS)

ppm 24.98 (T) ; 25.07 (T) ; 26.28 (T) ; 26.41 (T) ; 28.87 (T) ; 29.16 (T) ; 36.66 (T) ; 36.77 (T) ; 37.9 (T) ; 39.31 (T) ; 39.43 (T) ; 51.84 (D) ; 58.99 (S) ; 59.05 (S) ; 61 (T) ; 61.05 (T) ; 66.5 (T) ; 67 (D) ; 67.04 (T) ; 69.17 (D) ; 70.66 (D) ; 70.79 (D) ; 101.52 (D) ; 128.07 (D) ; 128.26 (D) ; 128.58 (D) ; 136.29 (S) ; 156.23 (S) ; 169.43 (S) ; 169.5 (S) ; 170 (S) 170.2 (S) ; 170.4 (S) ; 170.44 (S) ; 170.7 (S) ; 170.85 (S) ; 173.28 (S).

2.2.3 Réaction de déacétylation

3.746 ml d'eau et 0.254 ml (1.824 mmoles) de TEA sont ajoutés goutte à goutte à 220 mg (0.076 mmoles) de biantenne protégée dissous dans 4 ml de méthanol. Après 2 heures de réaction, le mélange est acidifié par 0.104 ml (1.824 mmoles) d'acide acétique glacial.

Après 15 min, la solution est évaporée à sec. Le résidu obtenu est remis en solution dans 100-150 ml d'eau et la solution lyophilisée.

les 138 mg récupérés après lyophilisation sont purifiés sur Biogel $P_4$ équilibré dans l'eau. Le produit est repéré par révélation à l'orcinol.

Le volume d'élution est de 230 ml.

Deux lyophilisations permettent d'obtenir 120 mg de biantenne déprotégée.

Rdt : 95 %

Masse : 1673 ($M^+$)

RMN$^1$H

$^{13}$C

RMN$^1$H ($D_2O$)

ppm 1.25 (m, 4H) ; 1.4 (m, 4H) ; 1.5 (m, 4H) 2.2 ( élargi, 4H) ; 2.5 (m, 2H) ; 2.7 (m, 1H) ; 3.15 (t élargi, 2H) ; 3.5 (dd, 6H) ; 3.6 (m, 12H) ; 3.75 (m, 6H) ; 3.93 (s élargi, 6H) ; 3.95 (d, 6H) ; 4.25 (d, 6H) ; 4.4 (d, 6H) ; 5.14 (s, 2H) ; 7.4 (s élargi, 5H).

RMN$^{13}$C ($D_2O$ - dioxane)

ppm 25.52 (T) ; 26.33 (T) ; 28.72 (T) ; 37.06 (T) ; 40.07 (T) ; 60.55 (S) ; 61.76 (T) ; 68.55 (T) ; 69.39 (D) ; 71.53 (D) ; 73.39 (D) ; 75.92 (T) ; 104.34 (D) ; 128.48 (D) ; 129.62 (D) ; 177.96 (S).

Masse (FAB) :

$M^+$ = 1672.7 (100 %);

$(M + 1)^+$ = 1673.8 (90 %) ;

$(M + 2)^+$ = 1674.8 (40 %).

2.3 Synthèse de la triantenne

2.3.1 Hydrogénolyse de la biantenne protégée

350 (0.134 moles) de Cbz Asp 6-amino Hex Tris tri (gal $Ac_4$)$_2$ sont ajoutés à 180 mg de palladium sur charbon en suspension dans 70 ml de mélange acide formique-eau (85/15). La solution est maintenue sous agitation et sous une pression en hydrogène de 30 psi pendant 16 h.

En fin de réaction, le catalyseur est éliminé par filtration sur membrane HVLP et le filtrat évaporé à sec.

Le résidu obtenu est remis en solution dans 100 ml de chloroforme. La phase organique est lavée 5 fois par 150 ml de $NaHCO_3$ à 5 % (p/v) dans l'eau, séchée, filtrée et évaporée.

Rdt : 92 %

2.3.2. Couplage entre le Asp 6-amino Hex Tris tri (gal $Ac_4$)$_2$ et l'ester activé de la monoantenne.

333 mg (0.226 mmoles) de monoantenne protégée et 32 mg (1.2 équivalents) de NHS sont dissous dans 10 ml d'acétate d'éthyle. la solution est amenée et maintenue à 4° C avant d'ajouter 1.2 équivalents (56 mg) de DCC.

Après 16 heures à 4° C, la dicyclohexylurée est éliminée par filtration sur membrane FH. Le filtrat est évapore à sec.

Les 0.149 mmoles de monoantenne ainsi activées sont directement mises en réaction avec 314 mg (0.123 mmoles) de Asp-[6-amino Hex Tris tri (gal $Ac_4$)]$_2$ dissous dans 15 ml d'acétonitrile. L'addition s'effectue sous atmosphère d'argon. La réaction se fait en présence de tamis moléculaire (8-12 mesh, Janssen 20860-4).

Après 63 heures à température ambiante, le milieu réactionnel est filtré sur membrane Millipore FH. Le filtrat est évaporé. Le résidu est remis en solution dans 100 ml de $CHCl_3$ et lavé 5 fois avec 150 ml d'eau. La phase organique est séchée, filtrée et évaporée.

Les 608 mg de produit brut obtenus sont purifiés par CLHP semi-préparatif par fractions d'environ 150 mg.

Dans le système éluant eau, acétonitrile (35/65), la tri antenne protégée élue après 33 min.

Les fractions qui la contiennent sont rassemblées et le mélange de solvants évaporés.

Après les différents passages, 116 mg sont récupérés.

Rdt :24 %

2.3.3 Réaction de déacétylation

4 ml d'eau contenant 1.7 mmoles de TEA sont ajoutés goutte à goutte à 116 mg (0.029 mmoles) de triantenne protégée dissous dans 4 ml de méthanol.

Après 2 heures, la réaction est complète (CCM - système A).

La solution est alors acidifiée en ajoutant 0.100 ml (1.79 mmoles) d'acide acétique glacial.

Après 15 min, la solution est évaporée à sec. Le résidu obtenu est remis en solution dans 100 ml d'eau puis lyophilisé. Les sels de triéthylamonium restants sont éliminés par filtration moléculaire : le produit de la lyophilisation est déposé sur une colonne de biogel $P_4$. La triantenne s'élue après 190 ml.

Nous obtenons après 2 lyophilisations, 70 mg (0.028 mmoles) de triantenne.

Rdt : 97 %

Masse : 2491 ($M^+$)

RMN

RMN$^1$H : ($D_2O$-dioxane à 3.70 ppm)

ppm 1.15 (massif), 1.44 et 1.53 (massif), 2.25 (massif), 2.6 (m), 3.13 (massif), 3.50 (dd), 3.65 (m), 3.70 (m + s), 3.90 ( s élargi), 3.95 (d), 4.26 (d), 4.37 (d), 4.74 (s), 5.14 (s), 7.14 (s élargi).

RMN$^{13}$C : ($D_2O$ - dioxane à 67.40)

ppm 25.48, 26.29, 28.70, 37.02, 37.81, 38.11, 40.13, 51.86, 52.97, 60.47, 61.70, 67.98, 68.54, 69.35, 71.49, 73.36, 75.85, 104.28, 128.37, 129.21, 129.57, 137.00, 157.52, 172.05, 172.35, 172.50, 173.40, 177.70.

## EXEMPLE 3

Propriétés physico-chimiques des différents composés

Dans le tableau 1 sont reprises les constantes physiques des composés : point de fusion, caractéristiques chromatographique ($T_R$, $R_f$, vol Elu. (volume d'élution)), masse moléculaire et rendement obtenus.

a. La composition des solvants utilisés en CCM est

A butanol-acide acétique-eau (4/1/1/)

B dichlorométhane-méthanol (9/1)

C hexane-chloroforme-acétone (10/9/1)

D chloroforme-acétone(8/2)

E butanol-acide acétique-pyridine-eau (15/3/10/12)

F acétonitrile-eau (6/4) (plaque $NH_2$)

G dichlorométhane-acétone (10/5)

La pureté des composés est entre autre vérifiée par CCM sur plaque de silicagel Merck 60F254 (Merck art 5735) ou sur plaque $NH_2$ dérivé de gel de silice (Merck art 15647).

b. La colonne utilisée est une microbondapack $C_{18}$ analytique : la phase mobile est $H_2O$ 0,05 % TFA/$CH_3CN$. Les proportions sont indiquées entre parenthèses.

c. Valeur déterminée sur biogel $P_4$ stabilisée dans $H_2O$

d. La colonne utilisée est une colonne carbohydrate analytique, la phase mobile est $H_2O$/$CH_3CN$ en proportion 7/3.

Tableau I

| Produits | Point de fusion pF (°C) | Rf (a) | Temps retention TR (min) (b) | Vol. Elution (ml) (c) | Rendement (%) |
|---|---|---|---|---|---|
| acide benzyloxycarbonamido-6-hexanoîque | 63 | 052 (A)<br>063 (B) | | | 89 |
| N-(tri(hydroxymethyl)methyl benzyloxy-carbonamido-6-hexanamide | 89-90 | 0.35 (A)<br>0.34 (B) | | | 56 |
| Bromure de tetra acétyl galactopyranosyle | 77-80 | 0.36 (C) | | | 80 |
| Cbz-6-amino Hex Tris tri ($galAc_4$) | | 0.66 (G) | 11.90 +/- 01 | | 23 |
| Cbz-Asp 6-amino Hex Tris tri $(galAc)_4$ (monoantenne protégée) | | 0.65 (F) | 3.91 +/- 0.02 (45/55) | | 91 |
| Cbz-Asp 6-amino hex Tris tri gal (monoantenne) | | 0.49 (F) | 16.90 +/- 01 (d) | 274 | |
| Cbz-Asp 6-amino hex Tris tri $(galAc)_{42}$ (biantenne protégée) | | 0.51 (G) | 10.15 +/- 0.03 (45/55) | | 46 |

Tableau I (suite)

| Produits | Point de fusion PF (°C) | Rf (a) | Temps rétention TR (min) (b) | Vol. Elution (ml) (c) | Rendement (%) |
|---|---|---|---|---|---|
| Cbz-Asp 6-amino Hex Tris tri $gal_2$ (biantenne) | | 0.65 (F) | | 230 | |
| Cbz-Asp 6-amino Hex Tris tri $(galAc)_{43}$ (triantenne protégée) | | 0.7 (A) | 22.66 +/- 0.05 (45/55) | | 24 |
| Cbz-Asp-Asp 6-amino hex Tris tri $gal_3$ (triantenne) | | 0.80 (F) | | 190 | |

**EXEMPLE 4**

1. Préparation de l'albumine humaine galactosylée (HSA gal)

Dix grammes de HSA (Mérieux, solution à 20 %) sont dissous dans un tampon borate de sodium 0.2 M pH 9, de façon à obtenir une concentration finale en protéine de 20 mg/ml.

Quarante grammes de lactose (monohydrate Merck art. 7660) sont alors ajoutés à la solution.

Lorsque tout est dissous, 25 g de Na B $H_3$ CN (Janssen 16.885.74) sont additionnés.

La solution est portée à 37° C et maintenue à cette température pendant 7 h. Elle est ensuite dialysée 2 fois dans 5 l d'acétate de sodium 0.1 M pH 4 puis 4 à 5 fois dans 5 l de PBS, ceci afin d'éliminer le lactose et le cyanobohydrure en excès.

La concentration en protéine est déterminée après la réaction, soit en mesurant la densité optique à 280 nm (DO 1 % = 5.8), soit en dosant les protéines par la méthode de Lowry avec de la BSA comme étalon.

Le nombre de galactoses couplés de façon covalente à la protéine se calcule à partir des données obtenues par dosage colorimétrique des sucres (§ 2 dosage) en utilisant du galactose comme étalon.

La solution de HSA gal est stérilisée par filtration sur membrane 0.22 µm (Millipore) et conservée à 4° C.

2. Dosages

2.1 Appareillage

Les densités optiques sont lues dans un spectrophotomètre de type UVIKON 610 CL (Van Hopplynus).

Les radiations β du tritium sont mesurées dans un compteur à scintillation liquide de modèle TRICARB 460 CD (Packard Inst. Comp. USA). Cet appareil tient compte des variations de l'efficience due à l'étouffement (Quenching) des solutions et calcule les résultats en désintégration par minute (dpm).

La radioactivité due à des radiations γ de l'iode 125 est mesurée dans un compteur gamma (L.K.B. Wallac 1272 Clinic gamma counter). Les résultats sont exprimés en coups par minute (cpm).

2.2 Dosage des hexoses (méthode de Dubois, 1956)

A 0.5 ml d'une solution de glycopeptide, de glycoprotéine ou de néoglycoprotéine correspondant approximativement à 25 µg d'hexoses, sont ajoutés, sous forte agitation, 13 µl d'une solution de phénol (Janssen 1.4.934.93) à 80 % (P/V) dans l'eau.

Ensuite, 1.250 ml d'acide sulfurique concentré sont additionnés. La solution prend une coloration jaune-orange qui est stable pendant plusieurs heures.

La densité optique est lue après 30 minutes à température ambiante, à 490 mm.

Une droite d'étallonage (5 à 50 µg) est effectuée pour le mannose (Merck art. 5984), le galactose (Merck art. 4058) et pour un mélange équimolaire de ces deux sucres.

2.3 Dosage des acides sialiques (méthode de Warren, 1959)

L'hydrolyse de l'acide sialique étant indispensable pour son dosage, 0.1 ml de solution de glycopeptide ou de glycoprotéine correspondant approximativement à 50 µg en acide sialique, est incubée pendant 1 h à 80° C en présence de 0.1 ml de $H_2SO_4$ 0.25 M.

Ensuite, 0.1 ml d'échantillon hydrolysé est prélevé et ajoouté à 50 µl de $NaIO_4$ (Merck art. 6597) 0.2 M dans du $H_3PO_4$9M. La solution est mélangée énergiquement puis laissé 20 min à température ambiante.

Cinq cents µl de Na As $O_2$ (Merck art. 6287) à 10 % (P/V) dans du $Na_2SO_4$ 0.5 M sont ajoutés. La solution est agitée jusqu'à décoloration complète. 1.5 ml d'acide thiobarbiturique (4.6 déhydroxy-2-mercaptopyrimidine Janssen D11.350.6) à 0.6 % (P/V) dans du $Na_2SO_4$ 0.5 M sont ajoutés. La mixture est homogénéisée et placée 15 min à 100 °C. Après 30 Min à température ambiante, le composé chromogène est extrait par 2.5 ml de cyclohexanone (Merck art. 2888). L'émulsion est centrifugée 3 min à 3000 g. La densité optique de la phase supérieure est lue à 549 nm.

Une droite d'étallonage (5 à 100 µg) est effectuée à partir d'acide sialique (Sigma type III A.3630).

2.4 Mesure de la radioactivité

la radioacivité due aux radiations β du tritium et associée aux glycopeptides, aux suspensions cellulaires, aux milieux de culture provenant des incubations de cellules à 4 °C et 37 °C, aux fractions subcellulaires (H, N, MLP, S) et aux fractions issues des gradients isocinétiques de saccharose de la fraction MLP, est déterminée après dispersion des échantillons dans 10 ml de mélange scintillant (Aqualuma, Lumac 3M Nederlands).

Les échantillons précipités au PTA sont neutralisés par un tampon phosphate avant d'être dispersés dans 15 ml de liquide scintillant.

Les fractions des gradients isocinétiques de densité élevée sont diluées dans de l'eau avant d'être mélangées au liquide scintillant.

Dans le cas des homogénats tissulaires, des aliquotes de 0.1 à 0.250 ml sont digérés dans 1 ml de Protosol (NEM, Dupont de Neumours, USA).

Les échantillons sont chauffés à 55 °C jusqu'à l'obtention d'une solution limpide (3 à 6 h).

Les échantillons sont ensuite décolorés par l'addition de 0.1 ml d'$H_2O_2$ à 30 % (V/V) (Merck art. 822287) puis chauffée 30 min à 55 °C.

Après correction du pH avec 0.1 ml d'acide acétique glacial, 10 ml de scintillant Biofluor (NEM, Dupont de Nemours, USA) sont additionnés.

Les échantillons sont mis à compter après 1 h.

Dans le cas du sang et des urines, 0.1 ml d'échantillon est ajouté à 0.5 ml d'un mélange Protosol-Ethanol (1/2 ; V/V). Après homogénéisation, les échantillons sont incubés 1 h à 55 °C puis décolorés en ajoutant, goutte à goutte, 0.5 ml d'HCl 0.5 N.

Les échantillons sont comptés après 24 h afin de réduire le bruit de fond au minimum (supérieur à 50 cpm).

Pour les solutions aqueuses, une courbe de mesure de l'étouffement est réalisée en utilisant le set standard tritié pour compteur à scintillation liquide (référence QCR-50, Amersham, Gakville, USA).

Pour les homogénats tissulaires, une courbe de mesure de l'étouffement est réalisée en utilisant des concentrations croissantes en protéines (0 à 60 mg) provenant de l'homogénéisation de l'ensemble des organes prélevés chez la souris et une concentration connue en standard tritié, n-hexadécane (référence TRR.6, Amersham, USA). Les échantillons sont préparés selon la méthode décrite pour les homogénats tissulaires.

Pour le sang, une courbe de mesure de l'étouffement est effectuée en utilisant des volumes croissants de sang (0 à 0.150 ml) et une concentration connue en standard tritié n-hexadécane. Les échantillons sont traités selon la procédure utilisée pour le sang et les urines.

La radioactivité due aux radiations de l'iode 125 et associée à la néoglyco-protéine (HSA gal), aux suspensions cellulaires, au milieu de culture, au sang, aux urines et aux organes est déterminée directement dans un compteur gamma.

3. Marquage radioactif

3.1 Marquage à l'iode 125 de l'HSA gal.

A 100 µg d'albumine humaine galactosylée dissous dans 50 µl de PBS sont ajoutés successivement 40 µl de tampon phosphate ($NaH_2PO_4$) 0.5 M pH 7.4, 1 mCi d'iode 125 (IMS 30-16.6 mCi/µg d'iode ; the Radiochemical Center, Amersham) et 15 µl d'une solution de PBS contenant 2 mg/ml de chloramine T.

Après 3 min à température ambiante, 15 µl d'une solution de PBS contenant 2.5 mg/ml de $Na_2S_2O_5$ - (UCB-Bruxelles) et 50 µl de sérum de veau foetal (Gibco) sont additionnés. L'HSA Gal rendue radioactive est séparée des composés de plus faible poids moléculaire par filtration moléculaire sur une colonne (1 X 30 cm) de Séphadex G 50 (Pharmacia -Medium 50-150 µm) équilibrée et élue par une solution de PBS à

un débit de 30 ml/heure.

La colonne est préalablement étalonnée avec de l'HSA Gal marquée au tritium. Son volume d'élution déterminé en mesurant la radioactivité associée aux différentes fractions est égal à 13.5 ml. Le profil d'élution de HSA Gal 125 I est déterminé par la mesure dans un compteur gamma de la radioactivité associée aux différentes fractions.

Le profil d'élution est superposable à celui de l'HSA $Gal^3H$. Les fractions contenant le pic de radioactivité sont rassemblées et la solution qui en résulte est stérilisée par filtration sur membrane de 0.22 µm (Acrodisc-Yelman).

Ce type de marquage radioactif permet d'obtenir des activités spécifiques de 6.5 à $7.10^6$ cpm/µg de protéine.

3.2 Marquage au tritium $^3H$ des glycopeptides

A 1 ml de solution stérile de tampon phosphate 0.1 M pH 7 contenant l'équivalent de 10 mg d'hexoses sont ajoutées 40 U d'enzyme Galactose Oxydase (activité spécifique : 100 U/mg de protéine-Sigma G 3385 type V, isolée à partir de Dactylium dendroïdes) dissoutes dans 400 µl de tampon phosphate 0.1 m pH 7, stérile.

La réaction se poursuit stérilement à 37° C pendant 24 heures. La solution est ensuite portée à pH 9 par du NaOH puis 50 à 100mCi de borohydrure de sodium tritié (Amersham-TRK 45 6.2-9.9 Ci/mmoles) dissous dans 100 µl de NaOH 10 mM sont ajoutés.

La réaction se poursuit pendant 1 h à 37° C ; ensuite, 1 mg de borohydrure de sodium non radioactif est ajouté à la solution ; après 10 min, la réaction est arrêtée par une goutte d'acide acétique glacial.

Le glycopeptide radioactif est séparé des produits de faible poids moléculaire par filtration moléculaire sur Biogel $P_4$. Le solvant d'élution est dans ce cas-ci de l'$H_2O$ milliQ.

Un dosage des hexoses et une mesure de la radioactivité permettent de localiser le composé. Les fractions le contenant sont rassemblées et la solution lyophilisée. Ce marquage radioactif sélectif permet d'obtenir des activités spécifiques élevées (7 à 8 $10^6$ dpm/µg d'hexose)

4. Marquage de la triantenne galactosylée au technecium biodistribution sur souris OEA

Toutes les solutions sont dégazées à l'azote. Les bouchons sont toujours fermés.

- 1 ml (2 mg) de Triantenne en solution dans l'eau,
- addition de 1,9 ml de NaCl 0,9 %,
- addition de 100 µl d'une solution de $SaCl_2$ à 1 mg/ml dans du HCl $2.10^{-2}$ M
- mesure du PH = 4,
- addition de 1 ml de $TcO_4^-$ (75 NaCl),
- après 30′, filtration (il reste 39 mCi) et TLC,
- 70 minutes plus tard, une deuxième série de TLC est effectuée.

**EXEMPLE 5**

Etude de l'interaction entre le vecteur hépatotrope et des hépatocytes en culture

L'étude *in vitro* des vecteurs hépatotropes de faible poids moléculaire a été effectuée sur des hépatocytes de rats isolés et mis en culture.

En fonction des conditions expérimentales, on a pu déterminer l'affinité relative des différents vecteurs pour le récepteur à galactoses présent à la membrane sinusoïdale des hépatocytes ;

Cette étude *in vitro* a été réalisée avec les mono, bi et tri-antennes synthétiques.

Affinités relatives

L'affinité relative a été déterminée par une expérience de compétition entre le vecteur et une néoglycoprotéine connue pour sa haute affinité pour les récepteurs à galactoses, soit l'albumine humaine

Expériences de compétition à 4°C

Afin de déterminer si les hépathocytes fixent spécifiquement les différents dérivés glycosylés (monoantenne = 3 galactoses terminaux ; biantenne = 6 galactoses terminaux ; triantenne = 9 galactoses terminaux), des expériences de compétition pour le récepteur à asialoglycoprotéines entre l'HSA gal et les différents composés ont été réalisées.

Des hépatocytes mis en culture depuis 16 h sont incubés à 4°C pendant 4 h dans 1 ml de milieu contenant une dose de 50 mg traceuse d'HSA gal marquée à l'iode-125 et des concentrations croissantes en glycopeptides selon l'invention non radioactifs.

Après le temps d'incubation, les cellules sont lavées, puis la radioactivité qui leur est associée, mesurée.

Les résultats présentés dans les figures 1 et 2 sont exprimés en pourcentage d'$^{125}$I HSA gal qui reste associé aux cellules en fonction du logarithme de la concentration molaire en compétiteur (figure 1) ou de la concentration molaire en galactoses du compétiteur (figure 2).

Les expériences montrent clairement que les concentrations en compétiteur nécessaires pour diminuer de 50 % la fixation de d'$^{125}$I HSA gal ($I_c$ 50) varient fortement en fonction du composé utilisé.

En effet, quelle que soit la concentration en monoantenne utilisés, celle-ci n'entre pas en compétition avec l'HSA gal iodiné pour le récepteur à asialoglycoprotéine présent à la membrane sinusoïdale des hépatocytes. Par contre, la présence de la biantenne ou de la triantenne dans le milieu d'incubation induit une diminution de la radioactivité associée aux cellules.

La valeur d'$I_c$ 50 (tableau ci-après) de la biantenne, bien que 600 fois inférieure à celle de l'HSA gal, indique que le composé est reconnu et se fixe aux récepteurs hépatiques.

La valeur d'$I_c$ 50 de la triantenne étant de 9 $10^{-8}$ M, son affinité est de l'ordre de 100 fois supérieure à celle de la biantenne.

Exprimée en concentration molaire en galactoses, l'affinité de la triantenne est du même ordre de grandeur que celle obtenue pour l'HSA gal.

Tableau 1

Inhibition de la fixation au récepteur à asialoglycoprotéine des hépatocytes de l'$^{125}$I HSA gal, par les différents composés galactosylés synthétisés (monoantenne = 3 galactoses terminaux ; biantenne = 6 galactoses term naux ; triantenne = 9 galactoses terminaux) et l'HSA gal.

*Détermination des concentrations molaires en compétiteur (A.) ou des concentrations molaires en galactoses du compétiteur (B.) nécessaires pour inhiber de 50 % la fixation de l'$^{125}$I HSA gal. La valeur obtenue pour l'HSA gal est indiquée comme référence.*

| COMPOSES GALACTOSYLES | A = $I_c$ 50 (molaire) | B = $I_c$50 (molaire en gal.) |
|---|---|---|
| Monoantenne | non déterminable | non déterminable |
| Biantenne | 7.6 $10^{-6}$ | 4.8 $10^{-5}$ |
| Triantenne | 9.1 $10^{-8}$ | 1.0 $10^{-6}$ |
| HSA gal | 1.2 AO$^{-8}$ | 3.3 $10^{-7}$ |

Conclusion

Les résultats obtenus montrent que l'affinité pour la lectine hépatique des composés synthétisés augmente en fonction du nombre de galactoses terminaux. Alors que la monoantenne possédant 3 galactoses terminaux se fixe aspécifiquement sur les cellules, la biantenne caractérisée par 6 galactoses en position terminale entre effectivement en compétition avec l'$^{125}$I HSA gal pour le récepteur.

Parmi les composés étudiés, c'est la triantenne qui a la plus haute affinité pour le récepteur. Elle possède en effet un pouvoir inhibiteur de la fixation de l'HSA gal radioactive par de l'HSA gal non radioactive.

Grâce à son affinité élevée pour le récepteur à asialoglycoprotéines, la triantenne ou composé à 9 résidus de galactoses terminaux que nous avons synthétisée, in vitro répond au critère primordial requis par les vecteurs hépatotropes.

## EXEMPLE 6

Distribution in vivo de la triantenne

Les résultats obtenus in vitro montrent que les hépatocytes de rat en culture fixent spécifiquement la triantenne. Afin de déterminer si ces données intéressantes peuvent être extrapolées in vivo, nous avons étudié la répartition tissulaire, sanguine, plasmique et urinaire de la triantenne administrée par voie intraveineuse à l'animal.

Distribution de la triantenne tritiée

Différentes concentrations en triantenne tritiée sont injectées dans la veine caudale des souris.

Les animaux sont sacrifiés 3 min après l'injection, et le sang, les différents tissus et les urines, prélevés.

Dans une première série d'expériences, des concentrations croissantes (4 à 3300 ng galactoses) en triantenne $^3H$ sont injectées dans la veine caudale de souris femelles OFI (Suisse) de 20 à 25 g. La dose que chaque animal reçoit est calculée à partir du poids de la seringue avant et après injection. Pour chaque concentration, trois souris de poids comparables sont sacrifiées, 3 min. après l'injection et de la façon suivante :

2 min. après l'injection, la souris est anesthésiée à l'éther ; 30 sec. plus tard, elle est placée et attachée sur une table à dissection et l'artère fémorale dégagée ; 3 min. précises après l'injection, l'artère fémorale est sectionnée.

Le sang prélevé à la pipette de pasteur héparinée (Héparine : S.I.D., S.A., Pharbil, Bruxelles - 1 ml = 5000 UI) est transféré dans des tubes en plastique contenant 10 µl d'une solution d'EDTA à 30 % dans l'eau. Le plasma est obtenu après 10 min. de centrifugation à 450 g (Heraeus -Christ, West Germany).

Les urines sont directement récoltées dans des tubes en plastique. Les différents organes (foie, rate, reins, coeur, poumons, muscles, cerveau, estomac, duodenum, colon, rectum) sont prélevés le plus rapidement possible, lavés dans une solution de PBS, séchés sur filtre, pesés puis homogénéisés dans 0.5 à 1 ml de PBS à 4° C à l'aide d'un Potter-Elvehjem en verre et d'un piston en teflon. Le système est ensuite rincé avec un volume identique de PBS à 4° C. Les suspensions sont ensuite soniquées 30 sec. à 50 W au sonicateur à sonde BRANSON B12.

La quantité de matériel radioactif associé au sang, au plasma, aux urines, aux suspensions tissulaires est déterminée comme décrit dans le § 2.4.

La concentration sanguine est calculée sur base correspondant à 7 % du poids corporel ; la concentration plasmatique sur base d'un volume correspondant à 3.5 % du poids corporel.

La quantité de matériel radioactif associé aux différents organes est reprise dans les figures 3 et 4 et est exprimée en pourcentage de la dose injectée (figure 3) ou en pourcentage de la dose injectée par gramme de tissu (figure 4). Il apparaît, d'une façon générale, que quelle que soit la dose de triantenne injectée, la quantité de matériel radioactif associé au sang ou au plasma est inférieure ou égale à 10 % de la dose initiale administrée.

Parmi les organes prélevés et quelles que soient les conditions expérimentales utilisées, c'est le foie qui renferme la plus forte concentration de triantenne.

La valeur associée au foie est, pour de faibles concentrations (0 - 100 ng galactoses), proportionnelle à la dose injectée et représente 45 % de ce qui a été administré.

Par contre, pour des concentrations plus élevées, ce pourcentage tend à diminuer (jusqu'à 3300 ng) et ne représente plus que 3 % pour une concentration de 100 µg en équivalents galactoses (figure 6a).

La quantité de vecteur lié au foie apparaît donc saturable en fonction de la dose administrée (figure 5).

La quantité de matériel associé aux reins, bien que 8 fois inférieure à celle liée au foie, est plus élevée que dans les autres organes et ceci jusqu'à une valeur injectée de 3300 ng.

Il est à noter toutefois, que dans le cas d'une concentration sursaturante de 100 µg, la valeur associée aux reins est de l'ordre de 3 à 4 fois plus élevée que celle liée au foie.

La radioactivité associée aux autres organes, comme la rate, le coeur, les poumons, les muscles, l'estomac, le duodenum, le colon, le rectum, est faible quelle que soit la quantité injectée, et sa valeur reste inférieure à 5 %. Ces mêmes résultats, exprimés par gramme de tissu, montrent clairement que la quantité de matériel lié à ces différents tissus diminue lorsque la concentration injectée augmente.

Distribution de la triantenne marquée au technetium

Une concentration saturante (100 µg galactose) de triantenne marquée au Technetium a été administrée par voie intra-veineuse à des souris (figure 6b). Dans ce cas, le pourcentage de radioactivité associé au foie est de l'ordre de 7 fois supérieur à celui du marquage au tritium.

Cette différence pourrait s'expliquer par la présence de colloïdes qui s'accumuleraient aussi dans le foie mais préférentiellement dans les cellules de Küpfer, cellules du système réticulo-endothélial caractérisés par leur pouvoir endocytaire important.

La quantité de matériel radioactif associé aux autres organes est également plus importante que dans le cas d'un marquage au tritium. Cette augmentation pourrait être expliquée par une déchélation du Technécium qui, une fois libéré, pourrait être capté de façon non spécifique par toutes les cellules. Des études préliminaires, in vitro, ont en effet montré que le dérivé technétié semblait peu stable.

L'excrétion rénale semble, dans ce cas-ci aussi, une voie d'élimination préférentielle du produit injecté.

Conclusions :

L'ensemble des résultats montre qu'après injection de faibles doses (jusqu'à 100 ng) de triantenne marquée au $^3H$, la majorité du matériel radioactif administré est associée au foie.

Des concentrations supérieures à 100 ng en équivalents galactoses saturent vraisemblablement les sites de reconnaissance du vecteur au niveau de la membrane des hépatocytes.

Tout surplus de vecteur n'ayant pas réagi avec le récepteur est éliminé par voie rénale plutôt que par une accumulation non spécifique dans les autres organes. En effet, quelle que soit la dose injectée, la rate, le coeur, le cerveau, le système digestif et les muscles n'accumulent que très peu de radioactivité.

Les données expérimentales présentées confirment d'une part les résultats obtenus in vitro, à savoir la haute spécificité du vecteur pour les hépatocytes et d'autre part, sa faible interaction avec la majorité des autres organes.

L'injection d'une faible quantité de vecteur semble toutefois préférable pour favoriser son accumulation préférentielle dans le foie.

L'excrétion urinaire de tout excédent est bénéfique car elle préserve les autres organes d'une possible toxicité secondaire.

Ces caractéristiques enrichies des paramètres pharmacocinétiques devraient permettre de moduler au mieux le mode d'administration du vecteur afin de transporter le plus sélectivement possible, des médicaments vers le foie.

**EXEMPLE 7**

**Résultats complémentaires et conclusions**

On a évalué ci-avant les potentialités d'une série de dérivés galactosylés de faible poids moléculaire en tant que transporteur d'agents actifs vers le foie et plus particulièrement vers les hépatocytes.

On a montré qu'il est possible de synthétiser des structures polygalactosylées complexes, à partir d'une unité de base, le Tris galactoside :

- le greffage d'une ou de deux unités Tris galactoside sur l'acide aspartique conduit aux dérivés monoantenne (3 galactoses terminaux) et biantenne (6 galactoses terminaux),
- la triantenne (9 galactoses terminaux) résulte d'un couplage entre un biantenne et une monoantenne.

La pureté des dérivés synthétisés, déterminée par chromatographie liquide à haute performance et sur base de la mesure de l'absorbance à la longueur d'onde de 210 nm, varie de 90 à 95 %.

Les structures des produits ont été contrôlées par RMN HI et C13 et par spectroscopie de masse et correspondent aux structures attendues.

L'incubation des différents dérivés de synthèse en présence d'hépatocytes de rat mis en culture montre que ni le Tris galactoside ni la monoantenne ne présentent d'affinité significative pour le récepteur d'Ashwell. Par contre, la biantenne et la triantenne possèdent une affinité importante pour la lectine hépatique. Ces expériences permettent, en outre, d'établir une relation entre l'affinité relative des composés pour le récepteur et le nombre de galactoses présents au sein de la molécule : triantenne > biantenne > > > monoantenne = Tris galactoside. L'affinité de la triantenne pour le récepteur est, 60 fois plus élevée que celle de la biantenne et pourrait dans ce cas ci, s'expliquer par une plus grande flexibilité du dérivé à 9 galactoses, ce qui aurait pour conséquence de favoriser l'orientation spatiale adéquate des galactoses, nécessaire pour la liaison du composé à la lectine hépatique.

Les études ultérieures, poursuivies uniquement avec le triantenne, ont permis de mettre en évidence 56370 et 490500 sites de respectivement, haute affinité (Kd = $1.7 \times 10^{-8}$) et de plus faible affinité (Kd = $3.1 \times 10^{-7}$).

A 37° C, les récepteurs intériorisent efficacement la triantenne. Par comparaison avec la capture de la macromolécule, ASH gal, les hépatocytes incubés pendant de courtes périodes (120 min), intériorisent jusqu'à 4 fois plus de molécules de triantenne.

Toutefois, après 27 h à 37° C en présence de $2.1 \times 10^{14}$ molécules de ligands (triantenne ou ASH gal), la capture des 2 composés galactosylés devient comparable et représente de l'ordre de $3.0 \times 10^{13}$ molécules par mg de PC. Ce fait pourrait s'expliquer par la formation préférentielle de complexes ligand-récepteur à vitesse de dissociation lente, dans le cas de la triantenne.

Les études de localisation subcellulaire de la triantenne indiquent qu'après fixation spécifique à la membrane plasmique des cellules, le composé galactosylé de synthèse est intériorisé puis transporté dans le compartiment lysosomial où il peut être dégradé. Le dérivé de synthèse est endocyté par un mécanisme similaire à celui utilisé par le glycopeptide naturel à 3 galactoses terminaux et la plupart des asialoglycoprotéines ou néoglycoprotéines.

In vivo, on a étudié la disparition sanguine ainsi que la distribution tissulaire de la triantenne suite à son administration intra-veineuse à des souris.

Bien que la triantenne soit éliminée très rapidement de la circulation sanguine et excrétée de façon significative dans les urines, on o observe une capture importante de la triantenne par le foie.

Les hépatocytes accumulent, en effet, 5 min après injection plus de 40 % de la quantité de triantenne administrée tandis que moins de 2 % de la triantenne interagissent avec les autres organes.

Exprimée par gramme de tissu (concentration spécifique), la concentration importante est toujours détectée dans le foie suivie par les reins. Les autres organes, tels que les poumons, la rate, le coeur et le système digestif en accumulent considérablement moins.

Les rapports des concentrations spécifiques du foie et des reins augmentent rapidement au cours du temps et passent de 1.5 à 10 après 10 min d'expérience, confirmant une accumulation plus spécifique par le foie.

Des études comparatives entre la triantenne et l'ASH gal permettent de mettre en évidence une vie plasmatique plus élevée pour la macromolécule (6 min), conséquence, entre autres d'une plus lente excrétion urinaire.

Cependant, après injection d'un nombre comparable de molécules, environ 2 fois plus de molécules de triantenne sont captées par le foie après un délai de 5 min.

Ces données confirment les résultats obtenus in vitro mettant en évidence une cinétique de fixation ou de capture différente pour les 2 types de composés galactosylés.

La confirmation, in vivo, des résultats mis en évidence in vitro, indique la validité du modèle des hépatocytes mis en culture en ce qui concerne l'interaction de la triantenne avec ce type cellulaire.

En résumé, on a montré qu'un dérivé polygalactosylé de faible poids moléculaire, la triantenne, se fixe au récepteur à gal/N-Acgal présent à la membrane sinusoïdale des hépatocytes puis est intériorisé par la cellule et a accès au compartement lysosomial où elle est lentement dégradée.

Bien que son administration intra-veineuse à des souris entraîne une excrétion urinaire significative, on a pu observé une accumulation spécifique et préférentielle dans le foie et plus particulièrement au sein des cellules parenchymateuses de cet organe.

Ainsi, le composé glycosylé à 9 galactoses terminaux synthétisé, en répondant aux critères de fixation et endocytose spécifiques avec accès aux lysosomes, peut être considéré comme vecteur hépatotrope lysosomotrope et en ce sens représente un vecteur sélectif dans notre concept de pilotage de substances médicamenteuses.

**EXEMPLE 8**

**IMAGERIE**

L'injection i.v. de triantenne marquée au technetium permet, d'une part d'obtenir rapidement une image du foie et d'autre part, d'étudier le fonctionnement du foie en déterminant le nombre de récepteurs à asialoglycoprotéines dans les cas normaux et pathologiques.

a) Imagerie du foie

L'utilisation de la triantenne marquée au technetium est particulièrement intéressante car elle permet la mise en évidence de lésions hépatiques. En effet, même si très localisées, ces lésions affectent l'image globale du foie et donc permettent un diagnostic précoce.

b) Etude fonctionnelle

La mesure de la radioactivité associée au foie permettrait de quantifier le nombre de récepteurs présents à la membrane sinusoïdale des hépathocytes et ainsi de différencier certains cas pathologiques de cas normaux. Certains troubles fonctionnels telle une diminution de la synthèse intracellulaire du récepteur entraîne une diminution du nombre de récepteurs exposés et par conséquent une diminution de la radioactivité associée au foie.

De même, toute perturbation de la glycosylation de la molécule constituant le récepteur, affecte sa capacité de fixer la triantenne.

D'autre part, la quantité de triantenne marquée associée au foie est dépendante de la vitesse de recyclage des récepteurs. Le recyclage étant lui-même dépendant du bon fonctionnement de la cellule hépatique en ce qui concerne l'acidification des vésicules d'endocytoses, la production d'ATP..., toute pathologie affectant ces différents paramètres devrait se répercutera sur l'image hépatique obtenue.

Ces différentes applications illustrent ainsi les possibilités d'utilisation de la triantenne marquée au technetium en imagerie hépatique.

## BIBLIOGRAPHIE

Ashwell G., Morell A.G., (1974). Adv. Enzymol. 41, 99-128.
Baenziger J.U., fiete D., J. of Biol. Chem. 254, 789-795.
Bodanszky M., Bodanszky A., dans : The PRACTICE OF peptide Synthesis. Springer-Verlag, Berlin heidelberg New York Tokyo.
Chin-Ping Yang and Chein-Shyong Su. (1986), J. Org. Chem. 51, 5186-5191.
Connolly D.T., Townsend R.R., Kawaguchi K., Bell W.R., Lee Y.C. (1982), J. of biol. Chem., 257, 939-945.
Ehrlich P., (1906), dans : Studies in Immunity, pp. 441, Wiley, N.Y.
Gregoriadis G; et Davis C. (1979), Biochem. Biophys. Res. Com., 89, 1287-1293.
hardy M.R., Townsend R.F., Parkhurst, S.M., Lee, Y.C. (1985). Biochemistry, 24, 22-28.
Kempen H.J.M. , Hoes C., van Boom J.H., Spanjer H.H., de Lange J., langendoen A. (1984), J. Med. Chem. 27, 1306-1312.
Lee, Y.C., Townsend R.F., Hardy M.R., Lönngren J., Arnap J., Haroldson M., Lönn H. (1983), J. of biol. Chem. 258, 199-202.
Masquelier, M. 1981 Thèse de doctorat à l'Universtié Catholique de Louvain : "Couplage covalent réversible de la daunorubicine et de la primaquine à des protéines : application à une chimiothérapie plus sélective du cancer et de la malaria.
Paulsen H., Schultz M. (1986), Liebigs Ann. Chem., 1435-1447.
Pirson P., 1984, Thèse de doctorat à l'Université Catholique de Louvain : "Mise au point de dérivés lysomotropes de la primaquine par association à des liposomes et par couplage à l'asialofétuine. Application à la chimiothérapie expérimentale de la malaria. (1979).
Stahl P.D., Rodman J.S., Miller P.J. et Schlesinger P.H., (1978), Proc. Nat. Acad. Sci. USA, 75, 1399-1403.
Stahl P.D. ET Schlessinger P.H. (1980), Trends in Biochem Sciences, 5, 1984-196.
Trouet A., Deprez-De Campeneere D., et De Duve C. (1972), Nature, New Biol., 239, 110-112.
Trouet A., Masquelier M., Baniani R. et Deprez-De Campeneere D. (1981); dans : Topics in Pharmaceutical

Sciences, Brumer D.D. et Speiser P. eds.-, pp. 153-162. Elsevier (North-Holland Biomedical press, Amsterdam.

## Revendications

1. Vecteur synthétique consistant dans un composé chimique de faible poids moléculaire, en particulier inférieur à 5000, présentant une affinité pour les cellules exposant à leur membrane plasmique des récepteurs reconnaissant spécifiquement des résidus sucrés consistant en des cycles glycosidiques de saccharides, caractérisé en ce qu'il présente une structure chimique hydrocarbonée de base sur laquelle sont fixés plusieurs cycles glycosidiques de saccharides espacés de telle manière que chaque site de reconnaissance de ces résidus sucrés du récepteur soit susceptible d'être occupé.

2. Vecteur synthétique selon la revendication 1, caractérisé en ce qu'il présente une structure chimique de base constituée par un acide aminé ou un peptide de 2 à 7 acides aminés, identiques ou différents avec au moins deux fonctions carboxyles et une fonction amine, libres, la ou certaines des fonctions(s) carboxyle libre(s) de l'acide aminé ou du peptide étant liée(s) par un lien amide à l'amine d'un groupe chimique doté en outre d'une fonction carboxyle, tel qu'un acide amino-alcanoïque, groupe servant de bras de liaison entre l'acide aminé et un groupe Tris, bras dont ladite fonction carboxyle est liée par un lien amide à l'amine du groupe Tris, ce dernier ayant certaines de ses fonctions hydroxy, transformées en fonctions éther par un reste de cycle glycosidique de saccharides déshydroxylé en position 1, de telle sorte que le vecteur dans son ensemble présente au moins autant de restes de cycles glycosidiques de saccharides que le récepteur n'a de site de reconnaissance de tels restes sucrés, et que ces restes de cycles glycosidiques de saccharides soient suffisamment espacés pour qu'une interaction puisse s'établir avec lesdits sites du récepteur.

3. Vecteur selon la revendication 2, caractérisé en ce que des restes hydrocarbonés divalents sont intercalés entre des restes de cycles glycosidiques et des fonctions hydroxyle des groupes Tris ainsi étherifiés par lesdits restes hydrocarbonés.

4. Vecteur selon la revendication 3, caractérisé en ce que les restes hydrocarbonés sont des restes d'acides aminés.

5. Vecteur synthétique selon l'une des revendications 2 à 4, caractérisé en ce que la ou une des fonctions amine libre(s) de l'acide aminé ou du peptide sont protégées par un groupe protecteur de fonction amine, tel que le groupe carboxybenzyloxy (Cbz).

6. Vecteur selon l'une des revendications précédentes, caractérisé en ce que ladite structure chimique de base consiste en un peptide comportant 2 ou 3 acides aminés.

7. Vecteur selon l'une des revendications précédentes, caractérisé en ce qu'il comporte 3 groupes Tris et 9 résidus sucrés.

8. Vecteur selon l'une des revendications 2 à 7, caractérisé en ce que le bras chimique entre une fonction carboxyle libre de l'acide aminé ou du peptide et un groupe Tris est un reste divalent d'un acide aminoalcanoïque de formule $-NH-(CH_2)_n-\underset{\underset{O}{\|}}{C}-$

avec n compris entre 2 et 12.

9. Vecteur selon l'une des revendications précédentes, caractérisé en ce que la structure chimique de base est constituée d'acide(s) aminé(s) comportant au moins un acide polyfonctionnel choisi parmi l'acide aspartique et l'acide glutamique.

10. Vecteur selon l'une des revendications précédentes, caractérisé en ce qu'il répond à une des formules développés suivantes :

Mono-antenne

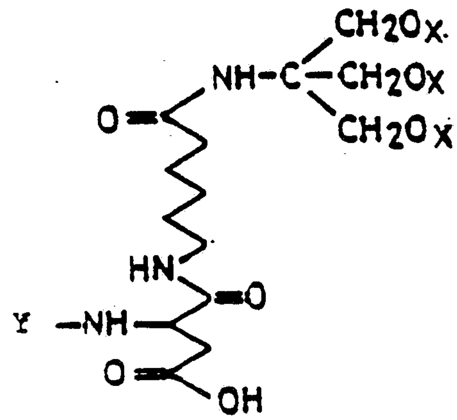

Bi-antenne

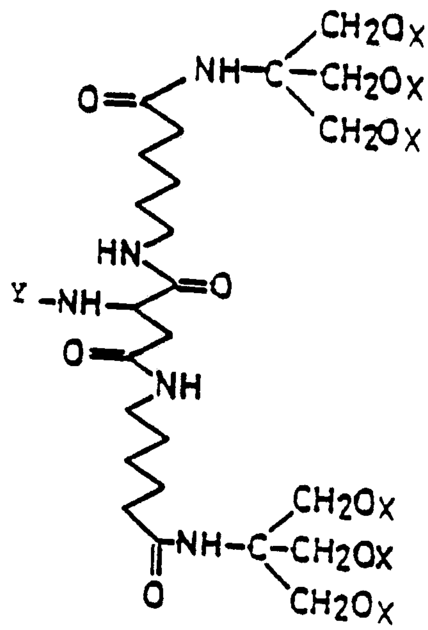

Tri-antenne

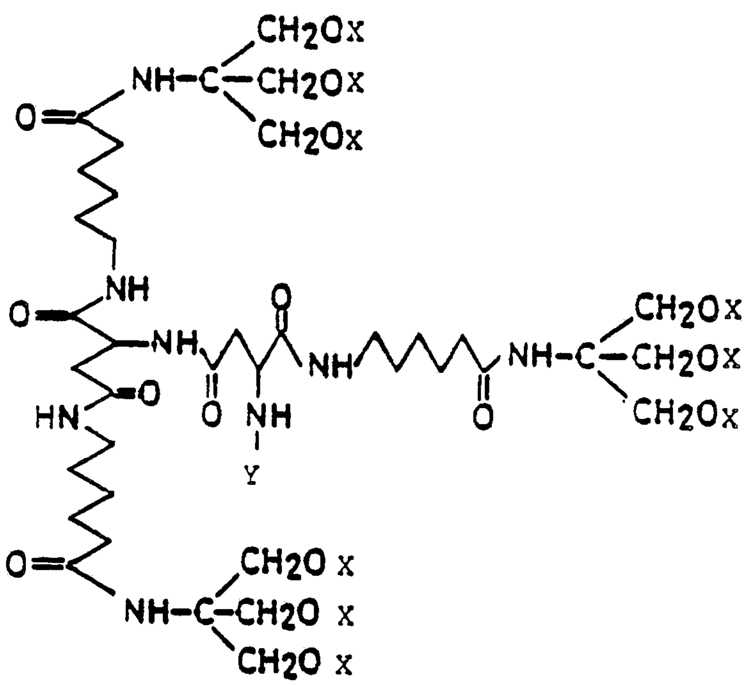

avec X identique ou différent = H ou un reste de cycles glycosydiques de saccharides dont les fonctions hydroxy sont éventuellement protégées, et

Y = H ou un groupe protecteur de la fonction amine tel que le groupe carboxybenzyloxy Cbz.

11. Vecteur selon l'une des revendications précédentes, caractérisé en ce que le reste de cycle de saccharides lié en sa position 1 sur les fonctions hydroxy des groupes Tris, ainsi sous forme d'éther, est choisi parmi un reste de

β-D-galactose

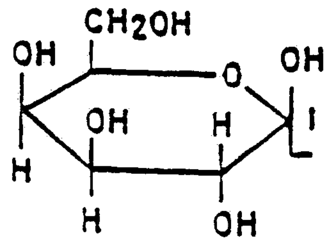

β-D-N-acetyl galactosamine

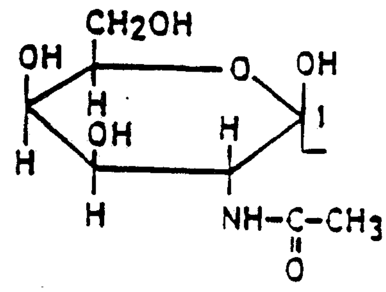

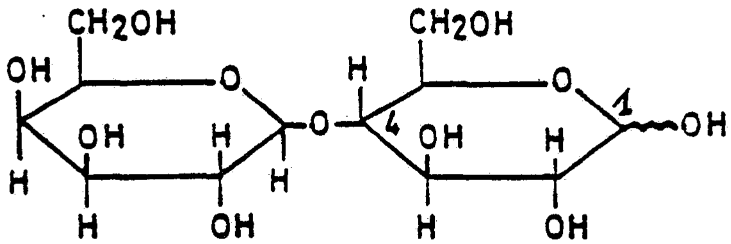

Lactose (4-O-β-D-galactopyranosyl-D-glucose)

ou
alpha D mannose

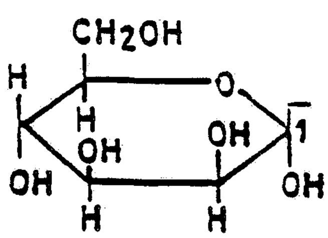

12. Vecteurs selon l'une des revendications 8 à 10, caractérisés en ce qu'un reste hydrocarbonée divalent est intercalé entre O et X, tel que le reste $-CH_2-CH_2-O-$.

13. Vecteur selon l'une des revendications précédentes, caractérisé en ce qu'il répond à l'une des formules générales suivantes.

Bi-antenne

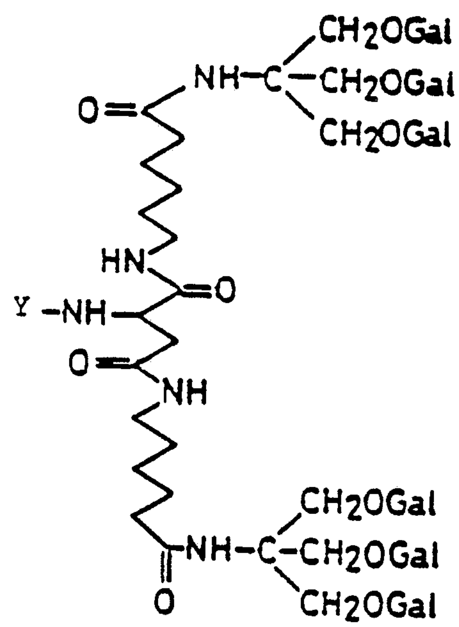

ou

Tri-antenne

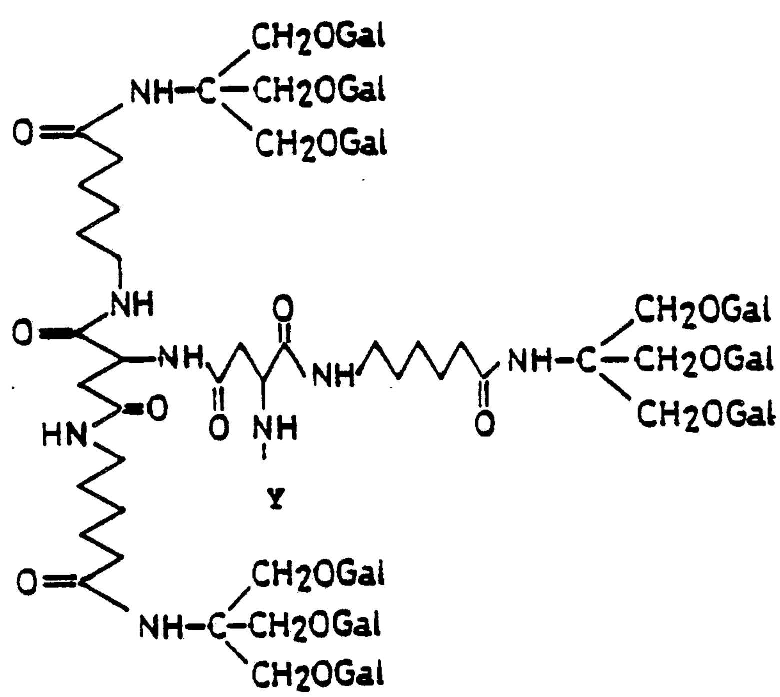

14. Utilisation d'un vecteur selon l'une des revendications 1 à 12 comme agent de pilotage en thérapie ciblée dans des conjugués du type vecteur-médicament ou vecteur-bras-médicament.

15. Conjugués selon la revendication précédente, caractérisés en ce que le médicament est choisi parmi les substances antitumorales, antivirales, et antiparasitaires.

16. Utilisation d'un vecteur selon l'une des revendications 1 à 12, à titre d'agent de pilotage à des fins d'imagerie médicale lorsque le vecteur est associé à un élément de marquage détectable, tel qu'une molécule radioactive.

17. Procédé de préparation d'un vecteur selon l'une des revendications 1 à 12, caractérisé en ce qu'on fixe plusieurs restes de cycles glycosidiques de saccharides sur une dite structure chimique hydrocarbonée.

18. Procédé de préparation de vecteur selon l'une des revendications 1 à 12, caractérisé en ce que l'on

effectue à partir d'un premier composé consistant en un groupe aminoalcanoïque dont la fonction amine est éventuellement protégée et dont la fonction carboxyle est liée à l'amine d'un groupe Tris, ce dernier ayant certaines de ses fonctions hydroxy étherifiées par des restes de cycles glycosidiques de saccharides déshydroxylés en position 1 dont les fonctions hydroxy sont protégées notamment par des groupes acétyle, les étapes suivantes :

- on restaure le cas échéant la fonction amine dudit groupe amino alcanoïque dudit premier composé par hydrogénation catalytique,
- on fait réagir ladite fonction amine avec une fonction carboxy libre d'un acide aminé ou d'un peptide de 2 à 7 acides aminés identiques ou différents, ayant au moins deux fonctions carboxy libres et une fonction amine protégée, pour obtenir un second composé,
- on déprotège les cycles glycosidiques dudit second composé notamment après déacétylation, pour obtenir un vecteur mono-antenne,
- un vecteur bi ou multi-antenne étant obtenu à partir d'un dit second composé selon qu'on couple une ou plusieurs fois avec une ou des nouvelle(s) molécule(s) identique(s) ou différentes d'un dit premier composé par un lien amide entre une ou des fonction(s) carboxylique restée(s) libre(s) de l'acide aminé ou du peptide dudit second composé, et la ou les fonctions amines du ou desdits premier(s) composé(s), pour obtenir un troisième composé,
- troisième composé qui une fois déprotégé sur ces cycles glycosidiques, notamment déacétylé, donne un vecteur bi ou multi-antenne.

19. Procédé selon la revendication précédente, caractérisé en ce qu'on couple une ou plusieurs fois un dit second composé avec ou plusieurs nouveau(s) dit(s) troisième(s) composé(s) identiques ou différents, par un lien amide entre une ou des fonction(s) carboxy libre(s) d'un dit second composé et la ou les fonctions amine libre(s) du ou desdit(s) troisième(s) composé(s).

FIG_1

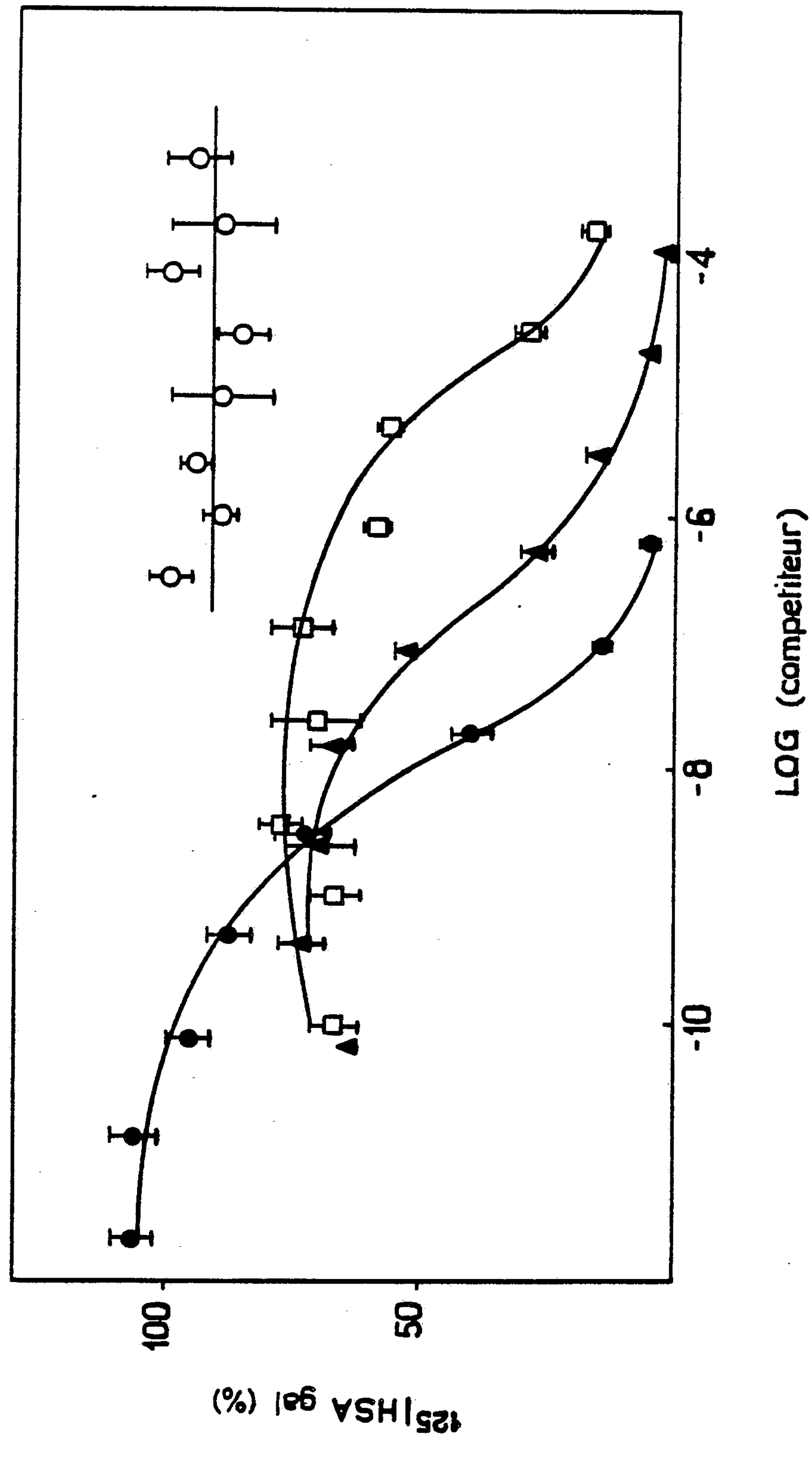

FIG. 2

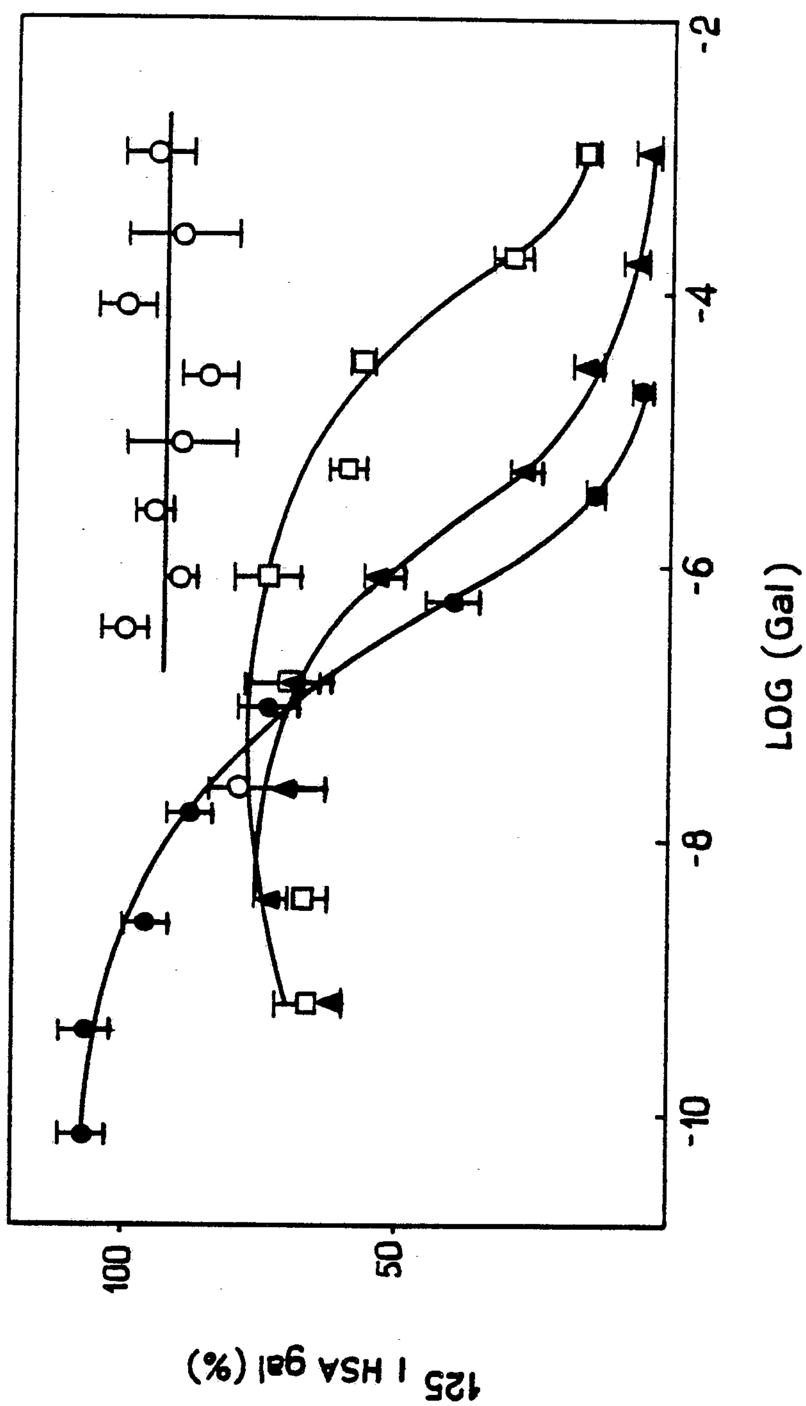

FIG_3

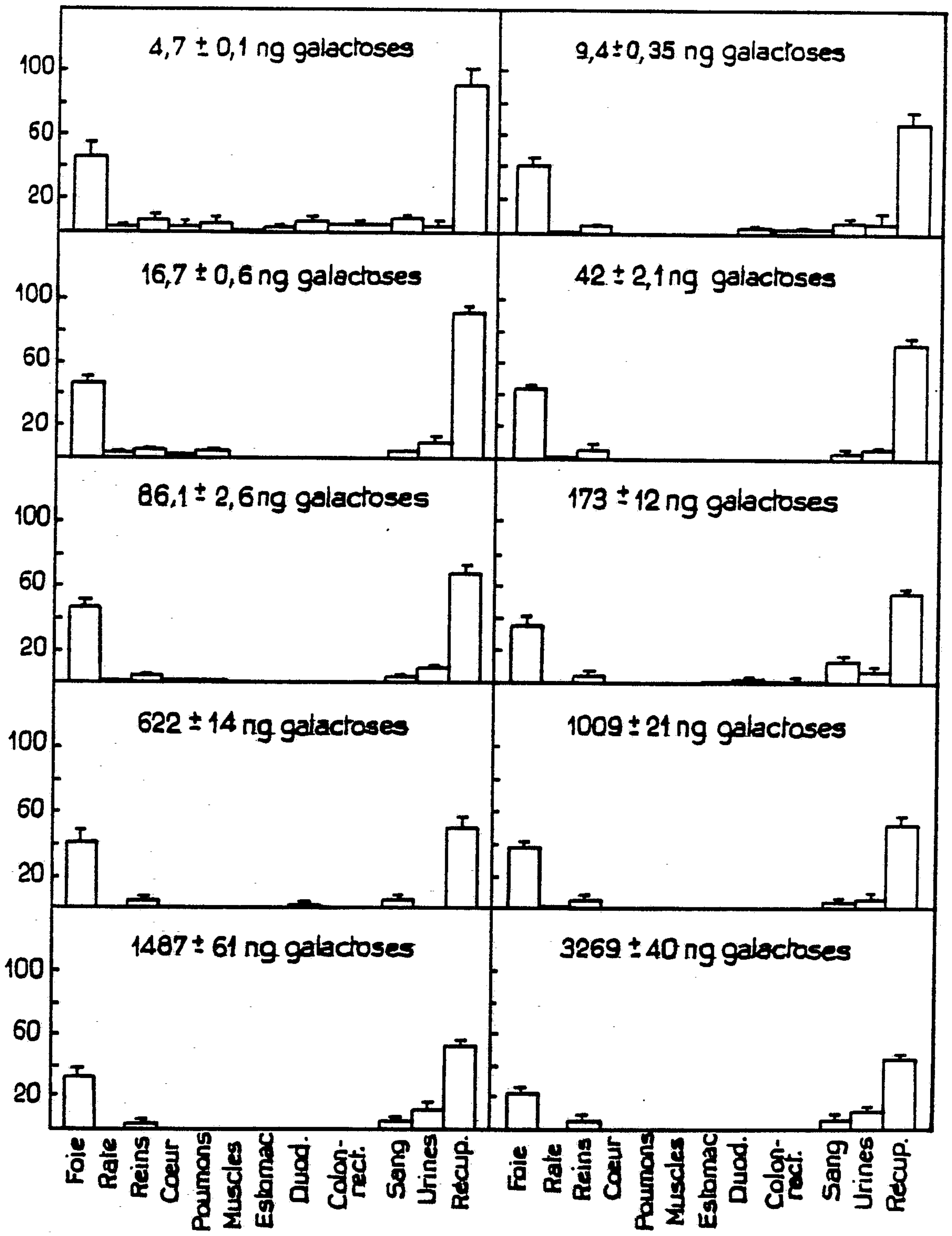
4,7 ± 0,1 ng galactoses
9,4±0,35 ng galactoses
16,7 ± 0,6 ng galactoses
42 ± 2,1 ng galactoses
86,1 ± 2,6 ng galactoses
173 ± 12 ng galactoses
622 ± 14 ng galactoses
1009 ± 21 ng galactoses
1487 ± 61 ng galactoses
3269 ± 40 ng galactoses
100
60
20
Foie
Rate
Reins
Coeur
Poumons
Muscles
Estomac
Duod.
Colon-rect.
Sang
Urines
Récup.

FIG_4

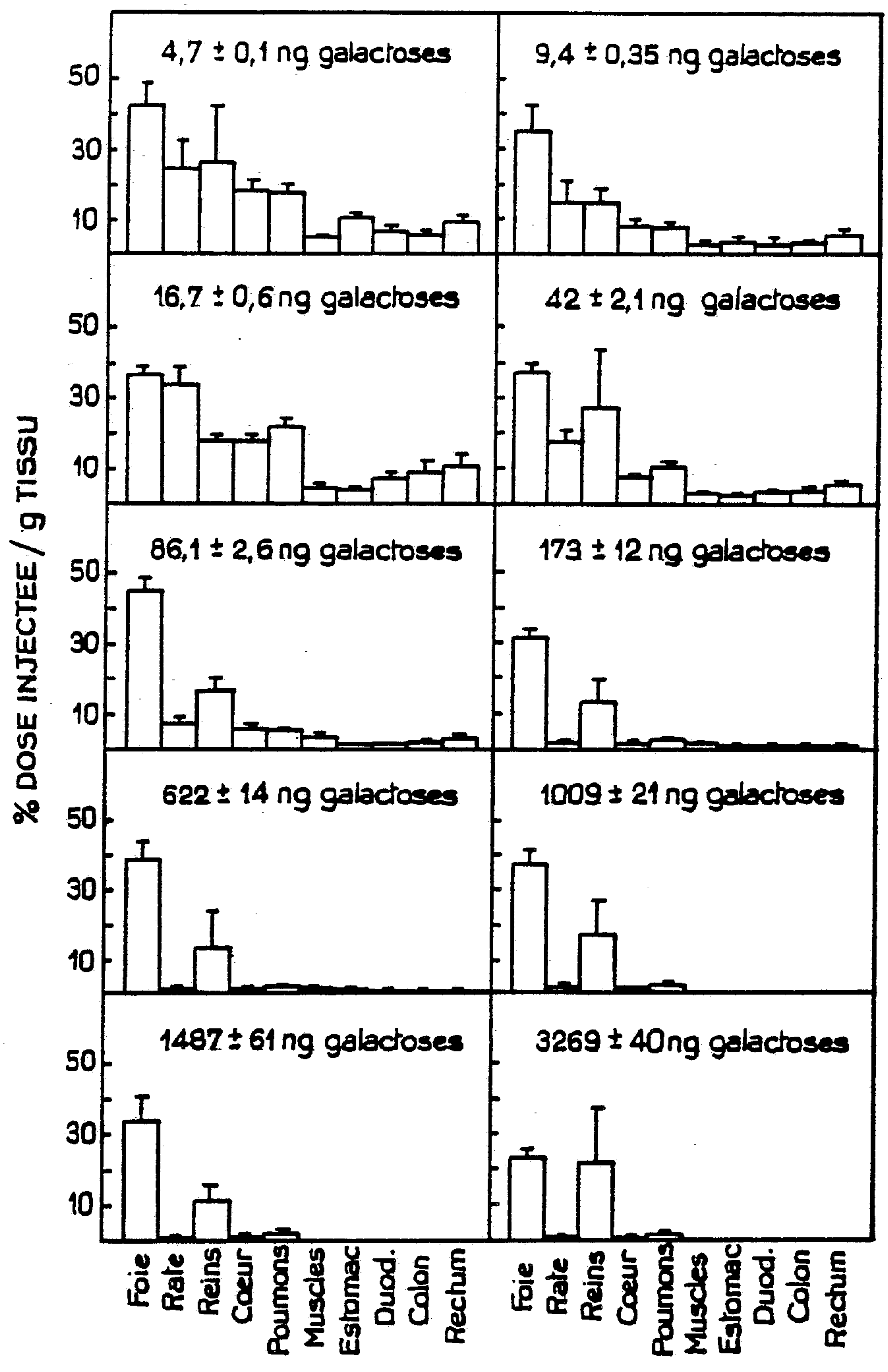
% DOSE INJECTEE / g TISSU
4,7 ± 0,1 ng galactoses
9,4 ± 0,35 ng galactoses
16,7 ± 0,6 ng galactoses
42 ± 2,1 ng galactoses
86,1 ± 2,6 ng galactoses
173 ± 12 ng galactoses
622 ± 14 ng galactoses
1009 ± 21 ng galactoses
1487 ± 61 ng galactoses
3269 ± 40 ng galactoses
50
30
10
Foie
Rate
Reins
Coeur
Poumons
Muscles
Estomac
Duod.
Colon
Rectum

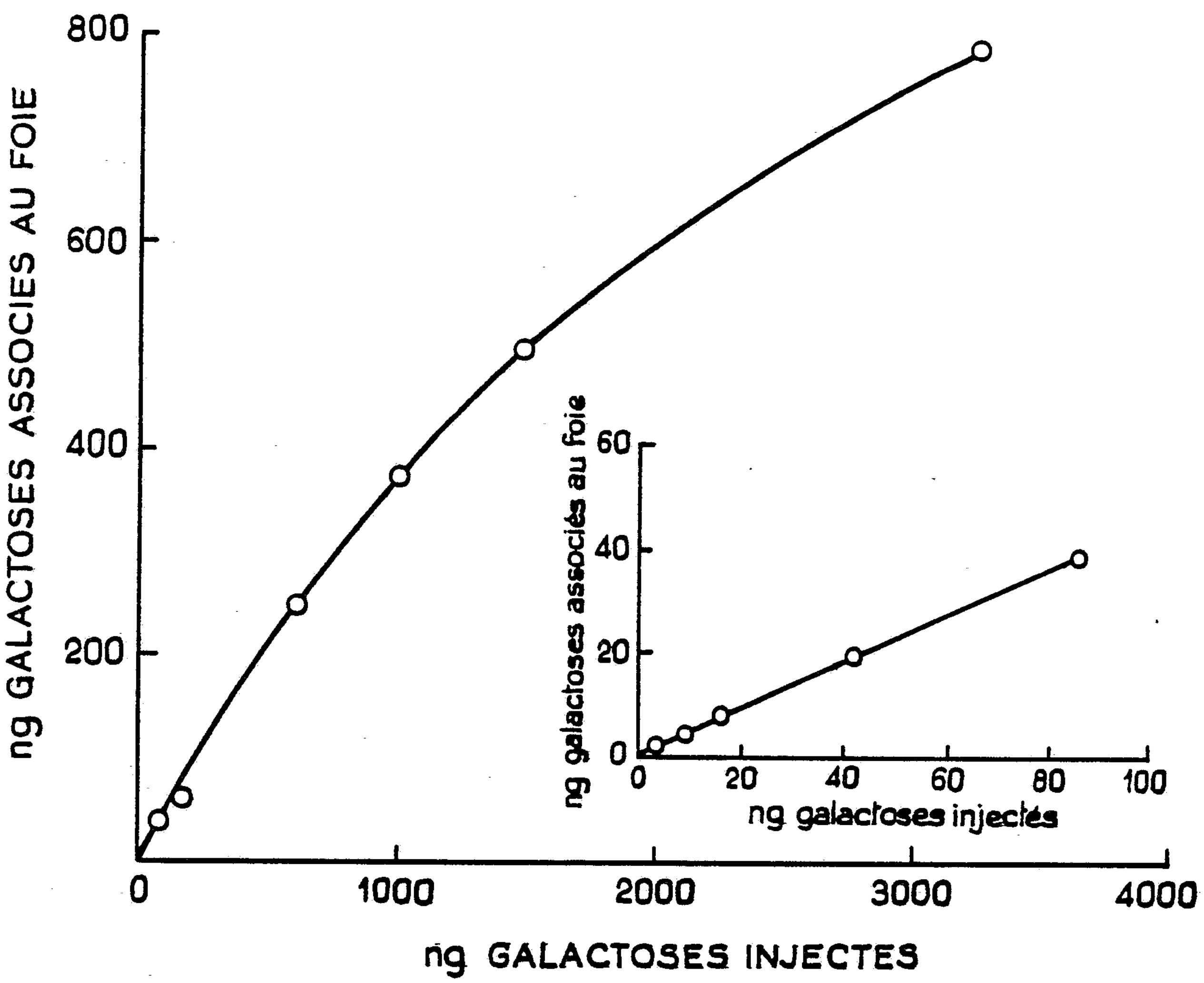
800
600
400
200
0
1000
2000
3000
4000
ng GALACTOSES ASSOCIES AU FOIE
ng GALACTOSES INJECTES
60
40
20
0
0
20
40
60
80
100
ng galactoses associés au foie
ng galactoses injectés

FIG_5

FIG_6

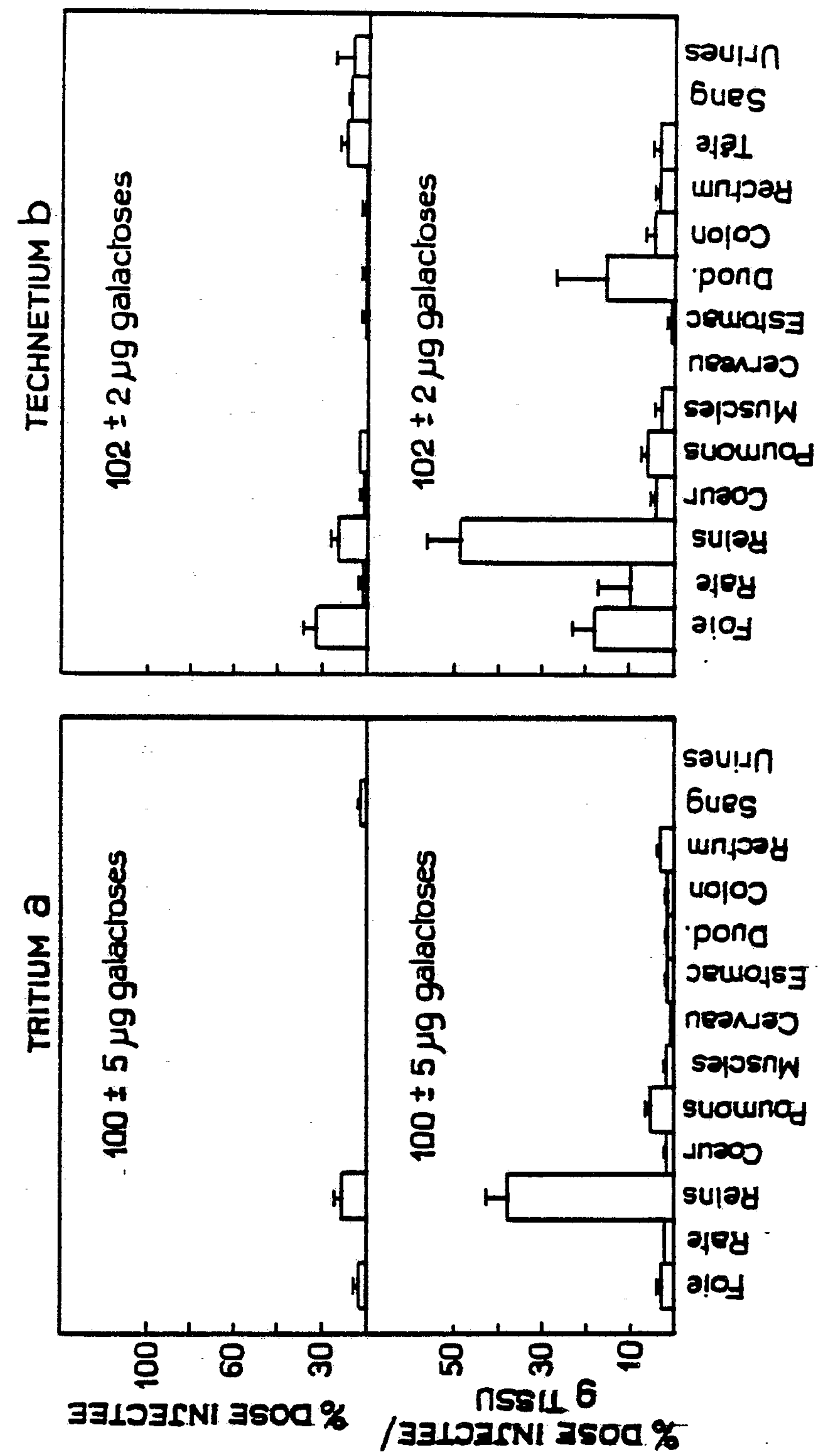
TRITIUM a
100 ± 5 µg galactoses
100 ± 5 µg galactoses
TECHNETIUM b
102 ± 2 µg galactoses
102 ± 2 µg galactoses
% DOSE INJECTEE
100
60
30
% DOSE INJECTEE/
g TISSU
50
30
10
Foie
Rate
Reins
Coeur
Poumons
Muscles
Cerveau
Estomac
Duod.
Colon
Rectum
Tête
Sang
Urines

Office européen des brevets

# RAPPORT DE RECHERCHE EUROPEENNE

Numero de la demande

EP 89 40 2730

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| X,Y | BIOCHEMISTRY, vol. 23, 1984, pages 4255-4261, American Chemical Society; R.T. LEE et al.: "New Synthetic cluster ligands for galactose/N-acetylgalactosamine-specific lectin of mammalian liver" * En entier * --- | 1-6,8-12,14 | C 07 H 15/04<br>A 61 K 47/00<br>A 61 K 49/02 |
| X,Y D | BIOCHEMISTRY, vol. 24, 1985, pages 22-28, American Chemical Society; M.R. HARDY et al.: "Different modes of ligand binding to the hepatic galactose/N-acetylgalactosamine lectin on the surface of rabbit hepatocytes" * En entier * --- | 1-6,8-12,14 | |
| Y,D | THE JOURNAL OF BIOLOGICAL CHEMISTRY, vol. 257, no. 2, 25 janvier 1982, pages 939-945, US; D.T. CONNOLLY et al.: "Binding and endocytosis of cluster glycosides by rabbit hepatocytes" * Résumé; page 940, diagram 1 * --- | 1-6,8-12,14 | **DOMAINES TECHNIQUES RECHERCHES (Int. Cl.5)** |
| A | ARCHIVES OF BIOCHEMISTRY AND BIOPHYSICS, vol. 260, no. 3, janvier 1988, pages 241-249, Academic Press, Inc.; Y. OHSUMI et al.: "Enhancement of macromolecular ligand binding by rabbit alveolar macrophages by mannose oligosaccharides and related compounds" * Résumé; page 242, diagram II * --- -/- | 1-6,8-12,14 | C 07 H 15/00<br>A 61 K 47/00<br>A 61 K 49/00 |

**Le présent rapport a été établi pour toutes les revendications**

| Lieu de la recherche | Date d'achèvement de la recherche | Examinateur |
|---|---|---|
| LA HAYE | 21-12-1989 | BRENNAN J. |

**CATEGORIE DES DOCUMENTS CITES**

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)

Office européen des brevets

# RAPPORT DE RECHERCHE EUROPEENNE

Numero de la demande

EP 89 40 2730

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| A | CARBOHYDRATE RESEARCH, vol. 67, 1978, pages 509-514, Elsevier Scientific Publishing Co., Amsterdam, NL; Y.C. LEE: "Synthesis of some cluster glycosides suitable for attachment to proteins or solid matrices"<br>* Page 509, lignes 1-14; page 513, composés 8-23 *<br>----- | 1-6,8-12,14 | |
| | | | DOMAINES TECHNIQUES RECHERCHES (Int. Cl.5) |

*Le présent rapport a été établi pour toutes les revendications*

| Lieu de la recherche | Date d'achèvement de la recherche | Examinateur |
|---|---|---|
| LA HAYE | 21-12-1989 | BRENNAN J. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant